# EUROPEAN PATENT SPECIFICATION

(11) **EP 2 571 569 B1**
(45) Date of publication and mention of the grant of the patent: **13.07.2016**
(21) Application number: 11783160.2
(22) Date of filing: 20.05.2011
(51) Int. Cl.: A61N 1/05, A61N 1/36

(54) **Encasement with an interior chamber for electrically insulating a conductor connector and an associated method of connecting leads**
Umhüllung mit einer inneren Kammer zur elektrischen Isolation eines Verbindungsstücks für Leiter und ein zugehöriges Verfahren zur Verbindung von Leitern
Enveloppe avec une chambre intérieure pour l'isolation électrique d'un connecteur de sondes et un procédé associé pour la connexion de sondes

(30) Priority: 21.05.2010 US 201013079318; 04.04.2011 US 201113079318
(43) Date of publication of application: 27.03.2013
(73) Proprietor: Cochlear Limited, Macquarie University, NSW 2109 (AU)
(72) Inventor: CRYER, Adrian, Robert, New South Wales 2109 (AU); MILIJASEVIC, Zoran, New South Wales 2109 (AU)
(74) Representative: Grünecker Patent- und Rechtsanwälte PartG mbB
(86) International application number: PCT/IB2011/052224
(87) International publication number: WO 2011/145084

(56) References cited:
- WO-A1-2010/028436
- US-A- 5 876 429
- US-A1- 2002 124 857
- US-A1- 2004 059 403
- US-A1- 2005 137 664
- US-A1- 2008 009 202
- US-A1- 2009 187 233
- US-A1- 2009 283 294
- US-B2- 6 921 295

## Description

### BACKGROUND

### Field of the Invention

The present invention relates generally to the formation of an implantable insulated electrical connection, and more particularly, to an implantable electrically insulated lead connector for electrically connecting lead(s) in an implantable medical device.

### Related Art

Medical devices having one or more implantable components, generally referred to herein as implantable medical devices, have provided a wide range of therapeutic benefits to patients (sometimes referred to herein as recipients) over recent decades. Included among implantable medical devices are active implantable medical devices (AIMDs), which are medical devices having one or more implantable components that rely for their functioning upon a source of power other than the human body or gravity, such as an electrical energy source. AIMDs often include an implantable, hermetically sealed electronics module and a device that interfaces with a patient's tissue, sometimes referred to as a tissue interface. The tissue interface may include, for example, one or more instruments, apparatuses, sensors or other functional components that are permanently or temporarily implanted in a patient. The tissue interface is used to, for example, diagnose, monitor and/or treat a disease or injury, or to modify a patient's anatomy or to modify a physiological process of a patient.

For example, an AIMD tissue interface may include one or more conductive electrical contacts, referred to as electrode contacts, which deliver electrical stimulation signals to, or receive signals from, a patient's tissue. The electrodes are typically disposed in a biocompatible electrically non-conductive carrier, and are electrically connected to the electronics module. The electrodes and the non-conductive member are collectively referred to herein as an electrode assembly.

An implantable medical device may also include multiple separate device components electrically connected to one another by leads. Leads extending between device components may be implanted along with the device components, and these leads may become damaged over time and require repair.

The document US 2004/059403 A1 discloses a sleeve for securing a pacer lead to adjacent tissue, the sleeve including an internal cavity to be filled with a saline solution.

The document WO 2010/028436 A1 discribes a sealable sleeve and a method of connecting leads using the sleeve.

### SUMMARY

An impervious encasement is disclosed. The impervious encasement is configured to electrically insulate a conductor connector configured to electrically connect distal ends of first and second leads electrically connected to first and second device components of an implantable medical device. The impervious encasement comprises a sleeve, configured to circumferentially surround the conductor connector, having an exterior wall and a deformable interior wall spaced to define a chamber therebetween.

An implantable medical device for implantation in a recipient's body is also disclosed. The implantable medical device comprises first and second elongate leads electrically connected to first and second device components of the implantable medical device, respectively, a conductor connector configured to electrically connect a distal end of the first lead to a distal end of the second lead and an impervious encasement electrically insulating the conductor connector. The impervious encasement comprises a sleeve, configured to circumferentially surround the conductor connector, having an exterior wall and a deformable interior wall spaced to define a first chamber therebetween.

A kit for connecting leads of implantable medical device components, is disclosed comprising first and second implantable components having first and second leads, respectively. The kit comprises a conductor connector configured to electrically connect distal ends of the first and second leads, a sleeve, configured to circumferentially surround the conductor connector, having an exterior wall and a deformable interior wall spaced to define a chamber therebetween and a fluent electrically insulative material configured to apply force to cause the interior wall to distend inwardly when the insulative material is disposed in the chamber.

A method of connecting leads of implantable medical device components, is further disclosed, the components comprising first and second implantable components having first and second leads, respectively. The method comprises electrically connecting distal ends of the first and second leads with a conductor connector, longitudinally displacing a sleeve along the first lead such that the sleeve circumferentially surrounds the conductor connector, wherein the sleeve has an exterior wall and a deformable interior wall spaced to define a chamber therebetween, and injecting a fluent electrically insulative material into the chamber such that the insulative material causes the interior wall to distend inwardly.

### BRIEF DESCRIPTION OF THE DRAWINGS

Illustrative versions of an implantable insulated lead connector are described herein with reference to the accompanying drawings, in which:
FIG. 1 illustrate an exemplary cochlear implant;
FIG. 2A is a perspective view of a portion of an exemplary internal component assembly of a cochlear implant, and a supplementary component that may be connected to the internal component assembly;
FIG. 2B is a perspective view of an internal component assembly of FIG. 2A electrically connected to the supplementary component of FIG. 2A via an implantable insulated lead connector;
FIG. 2C is a perspective view of a portion of an exemplary internal component assembly of a cochlear implant;
FIG. 2D is a perspective view of a portion of an exemplary internal component assembly of a cochlear implant having a partly helixed lead;
FIG. 2E is a more detailed perspective view of an unhelixed region of the partly helixed lead illustrated in FIG. 2D;
FIGS. 3A-3D are side views illustrating an exemplary process for connecting leads of respective device components of an implantable medical device using an implantable insulated lead connector;
FIG. 3E is a perspective view of a longitudinally split sleeve of an implantable insulated lead connector;
FIGS. 4A-4D are side views illustrating an exemplary process for connecting leads of respective device components of an implantable medical device using an implantable insulated lead connector;
FIG. 4E is a cross-sectional view of the implantable insulated lead connector of FIG. 4D;
FIG. 5A is a cross-sectional view of portions of an implantable insulated lead connector configured to form multiple electrical connections between leads of respective device components of an implantable medical device;
FIGS. 5B-5E are side views illustrating an exemplary process for connecting leads of respective device components of an implantable medical device using the implantable insulated lead connector of FIG. 5A;
FIG. 6A is a perspective view of components of an implantable insulated lead connector ;
FIG. 6B is a cross-sectional view of components of an implantable insulated lead connector;
FIG. 6C is a side view of an implantable insulated lead connector;
FIGS. 7A-7C are side views illustrating an exemplary process for connecting leads of respective device components of an implantable medical device using an implantable insulated lead connector; and
FIGS. 8A-8C are side views illustrating an exemplary process for connecting leads of respective device components of an implantable medical device using an implantable insulated lead connector;
FIG. 9A is a cross-sectional view of a sleeve of an implantable insulated lead connection in accordance with embodiments of the present invention;
FIG. 9B is a perspective view of a female screw connector of an implantable insulated lead connection in accordance with an embodiment of the present invention;
FIG. 9C is a cross-sectional view of the female screw connector of FIG. 9B;
FIG. 9D is a perspective cross-sectional view of a screw connector of an implantable insulated lead connection in accordance with an embodiment of the present invention;
FIGS. 9E and 9F are cross-sectional views illustrating a process for forming an implantable insulated lead connection in accordance with an embodiment of the present invention;
FIG. 9G is a perspective view of an implantable insulated lead connection in accordance with an embodiment of the present invention;
FIG. 9H illustrates an implantable insulated lead connector in accordance with another embodiment of the present invention; and
FIG. 9I illustrates an implantable insulated lead connector in accordance with yet another embodiment of the present invention.

### DETAILED DESCRIPTION

An implantable electrically insulated lead connector is described that electrically connects components of an implantable medical device. The implantable insulated lead connector comprises a conductor connector electrically connecting conductors of two leads, and an ionically impervious encasement formed around the conductor connector. The impervious encasement is formed by a sleeve positioned around the conductor connector and a fluent electrically insulative material conformed around the conductor connector in a space between the conductor connector and the sleeve. Advantageously, the impervious encasement substantially prevents the ingress of electrically conductive body fluid and tissue to prevent the formation of any substantial conductive path of electrically conductive body fluid and/or tissue from the conductor connector into or out of the impervious encasement, and thereby insulates the conductor connector. In certain cases, the electrically insulative material utilized to form the impervious encasement may be a curable insulative material. In such cases, after filling the space between the conductor connector and the sleeve with the curable insulative material, the curable insulative material may be cured in situ (e.g., cured at, proximal to or adjacent to the site of implantation of the implantable insulated lead connector).

Implantable insulated lead connectors can provide electrical connections having superior reliability and efficiency by substantially preventing the ingress of electrically conductive body fluid and tissue. For example, by insulating the conductor connector with the impervious encasement, an implantable insulated lead connector is provided that may reduce leakage current and power loss at the site of an electrical connection relative to conventional connectors that attempt to seal a potential pathway for electrically conductive body fluid and tissue via the compression of separate device components against one another.

Exemplary implantable insulated lead connectors are described herein with reference to one type of implantable medical device, namely, a cochlear implant. It would be appreciated that an implantable electrically insulated lead connector may be used in other implantable medical devices. For example, implantable devices in which the connectors may be implemented include, but are not limited to, implantable medical devices such as neural stimulators, pacemakers, fluid pumps, sensors, drug delivery systems and other prosthetic hearing devices. It would also be appreciated that an implantable insulated lead connector may be used to connect a variety of different device components. For example, the implantable insulated lead connector may be used to connect an auxiliary power source or a microphone to another device component.

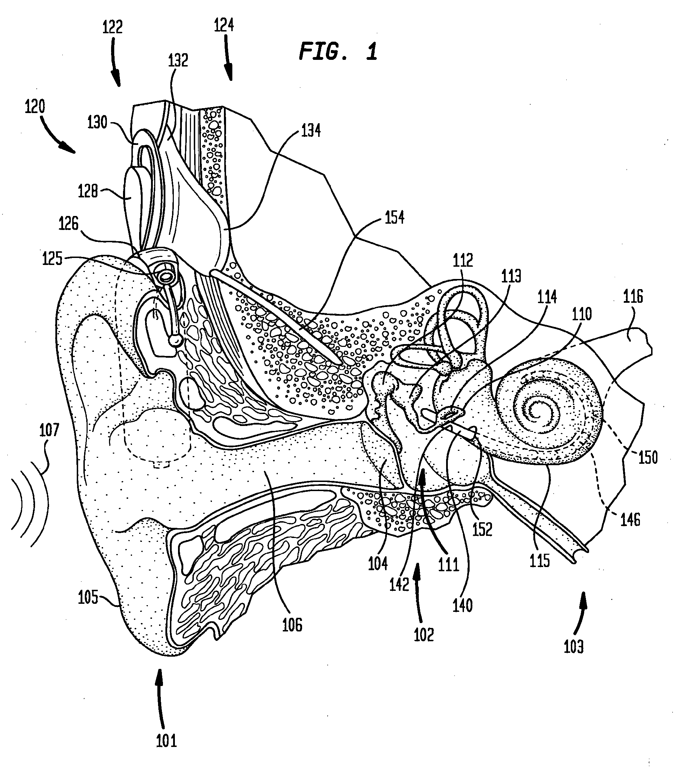

FIG. 1 illustrates an exemplary cochlear implant. In a fully functional human hearing anatomy, outer ear 101 comprises an auricle 105 and an ear canal 106. A sound wave or acoustic pressure 107 is collected by auricle 105 and channeled into and through ear canal 106. Disposed across the distal end of ear canal 106 is a tympanic membrane 104 which vibrates in response to acoustic wave 107. This vibration is coupled to oval window or fenestra ovalis 110 through three bones of middle ear 102, collectively referred to as the ossicles 111 and comprising the malleus 112, the incus 113 and the stapes 114. Bones 112, 113 and 114 of middle ear 102 serve to filter and amplify acoustic wave 107, causing oval window 110 to articulate, or vibrate. Such vibration sets up waves of fluid motion within cochlea 115. Such fluid motion, in turn, activates tiny hair cells (not shown) that line the inside of cochlea 115. Activation of the hair cells causes appropriate nerve impulses to be transferred through the spiral ganglion cells and auditory nerve 116 to the brain (not shown), where they are perceived as sound. In certain profoundly deaf persons, there is an absence or destruction of the hair cells. Cochlear implants, such a cochlear implant 120, are utilized to directly stimulate the ganglion cells to provide a hearing sensation to the recipient.

FIG. 1 also illustrates the positioning of cochlear implant 120 relative to outer ear 101, middle ear 102 and inner ear 103. Cochlear implant 120 comprises external component assembly 122 which is directly or indirectly attached to the body of the recipient, and an internal component assembly 124 which is temporarily or permanently implanted in the recipient. External assembly 122 comprises microphone 125 for detecting sound which is output to a behind-the-ear (BTE) speech processing unit 126 that generates coded signals which are provided to an external transmitter unit 128, along with power from a power source (not shown) such as a battery. External transmitter unit 128 comprises an external coil 130 and, preferably, a magnet (not shown) secured directly or indirectly in external coil 130.

In the cochlear implant illustrated in FIG. 1, internal component assembly 124 comprises an internal coil 132 of a stimulator unit 134 that receives and transmits power and coded signals received from external assembly 122 to other elements of stimulator unit 134 which apply the coded signal to cochlea 115 via an implanted electrode assembly 140. Connected to stimulator unit 134 is a flexible cable 154. Flexible cable 154 electrically couples stimulator unit 134 to electrode assembly 140. Electrode assembly 140 comprises a carrier member 142 having one or more electrodes 150 positioned on an electrode array 146. Electrode assembly 140 enters cochlea 115 at cochleostomy region 152 and is positioned such that electrodes 150 are substantially aligned with portions of tonotopically-mapped cochlea 115. Signals generated by stimulator unit 134 are typically applied by the array 146 of electrodes 150 to cochlea 115, thereby stimulating auditory nerve 116.

Although implantable insulated lead connectors are described herein with reference to a cochlear implant 120 having external and internal components, it would appreciated that connectors may also be implemented in a totally implantable cochlear implant. In such totally implantable devices, the sound processor and/or the microphone may be implanted in the recipient. Such totally implantable devices are described in, for example, H. P. Zenner et al. "First implantations of a totally implantable electronic hearing system for sensorineural hearing loss", in HNO Vol. 46, 1998, pp. 844-852; H. Leysieffer et al. "A totally implantable hearing device for the treatment of sensorineural hearing loss: TICA LZ 3001", in HNO Vol. 46, 1998, pp. 853-863; and H. P. Zenner et al. "Totally implantable hearing device for sensorineural hearing loss", in The Lancet Vol. 352, No. 9142, page 751.

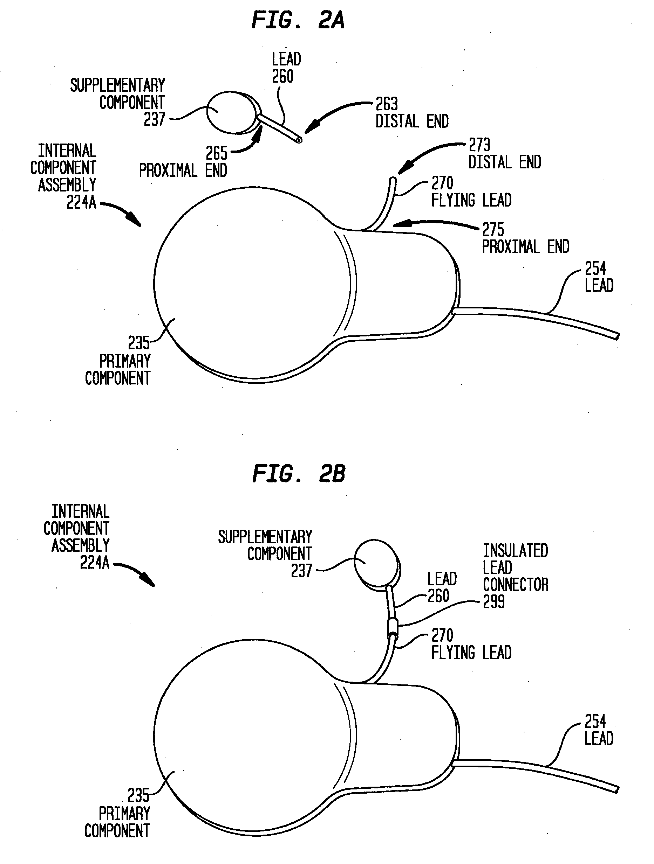

FIG. 2A is a perspective view of a portion of an exemplary internal component assembly 224A of an implantable medical device, namely a cochlear implant, and a supplementary component 237 that may be connected to the internal component assembly 224A. As illustrated in FIG. 2A, internal component assembly 224A, which is similar to internal component assembly 124 of FIG. 1, comprises a primary component 235 having a lead 254 that extends from primary component 235 to an electrode assembly (not shown), such as electrode assembly 140 of FIG. 1. In the illustrative case of FIG. 2A, primary component 235 is similar to stimulator unit 134 of FIG. 1 and is fully functional without supplementary component 237. Primary component 235 is implanted with a lead 270 having a proximal end 275 connected to primary component 235 and a distal end 273 that is not connected to any other module. In the illustrative case of FIG. 2A, lead 270 is a flying lead, and is completely insulated when implanted with primary component 235. As used herein, a 'flying lead' is a lead that, when implanted, is connected at a first end to an implantable component of an implantable medical device and that is not connected to any other component at a second end. A flying lead may be used to connect a primary component to a supplementary component via a post-manufacture connection procedure utilizing an implantable insulated lead connector.

Supplementary component 237 of FIG. 2A comprises a lead 260 having a proximal end 265 connected to supplementary component 237 and a distal end 263 that is not connected to any other component when manufactured. FIG. 2B is a perspective view of an internal component assembly 224A electrically connected to a supplementary component 237 via an implantable electrically insulated lead connector 299. In the illustrative case of FIGS. 2A and 2B, flying lead 270 extends from primary component 235 and is not connected to any other component at distal end 273 when initially implanted. During a subsequent surgical procedure to implant supplementary component 237, primary component 235 is electrically connected to supplementary component 237 by electrically connecting leads 260 and 270 via implantable insulated lead connector 299.

In some cases, supplementary component 237 is an upgrade module. In such cases, the upgrade module may be connected to primary component 235 to provide additional functionality to primary component 235. Providing additional functionality via an upgrade module is advantageous because the additional functionality may be provided without replacing primary component 235 and other components connected to it, such as an electrode assembly, for example.

In other cases, supplementary component 237 is a repair module. In such cases, when a primary component 235 malfunctions, a repair module is connected to primary component 235 to provide internal component assembly 224A with the capabilities lost due to the malfunction. Providing the lost capabilities via a repair module is advantageous because repairing the cochlear implant may be accomplished without replacing primary component 235 and other components connected to it, and without explanting primary component 235 for repairs.

Accordingly, providing one or more flying leads 270 extending from primary component 235 allows internal component assembly 224A to be upgraded and/or repaired via upgrade and repair modules with less invasive surgery than would be required to replace a complete cochlear implant. Such upgrades and repairs are also less surgically invasive than explanting primary component 235 for repairs or replacing primary component 235 and other components connected to it, such as an electrode assembly. An upgrade or repair module may be connected to a primary component via an implantable insulated lead connector that substantially prevents the ingress of electrically conductive body fluid or tissue to prevent the formation of any substantial conductive path of body fluid and/or tissue out of the connector.

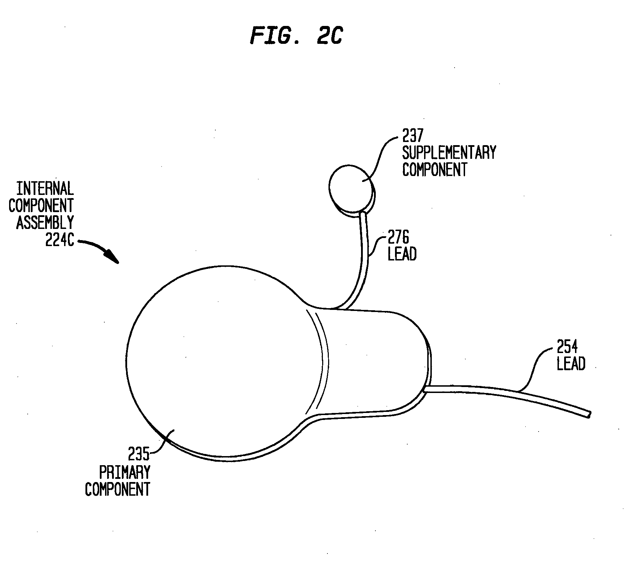

FIG. 2C is a perspective view of a portion of an exemplary internal component assembly 224C of a cochlear implant. Primary and supplementary components 235 and 237 are similar to those described with regard to FIGS. 2A and 2B, except that they are electrically connected by a lead 276 when manufactured, and are implanted together. Primary and supplementary components 235 and 237 may be referred to as distributed implantable components of internal component assembly 224C. In such cases, supplementary component 237 may provide additional or redundant functionality to primary component 235. As described further below, implantable insulated lead connectors may be advantageous for repairing the connection between primary and supplementary components 235 and 237. The connection may require repair when, for example, one or more conductors of lead 276 become exposed or a break in lead 276 occurs. Lead 276 may be repaired using an implantable insulated lead connector 299. A lead 276 repaired using implantable insulated lead connector 299 will result in an electrical connection of primary and supplementary components 235 and 237 similar to that shown and described in relation to FIG. 2B.

Alternatively, implantable insulated lead connectors may be used to connect primary component 235 to a replacement component. For example, to replace supplementary component 237, lead 276 may be severed and a replacement component may be connected to the portion of lead 276 extending from primary component 235. More specifically, a lead extending from the replacement module may be electrically connected to the portion of lead 276 extending from primary component 235 using an implantable insulated lead connector 299, similar to the manner in which leads 260 and 270 are connected via implantable electrically insulated lead connector 299 as illustrated in FIG. 2B.

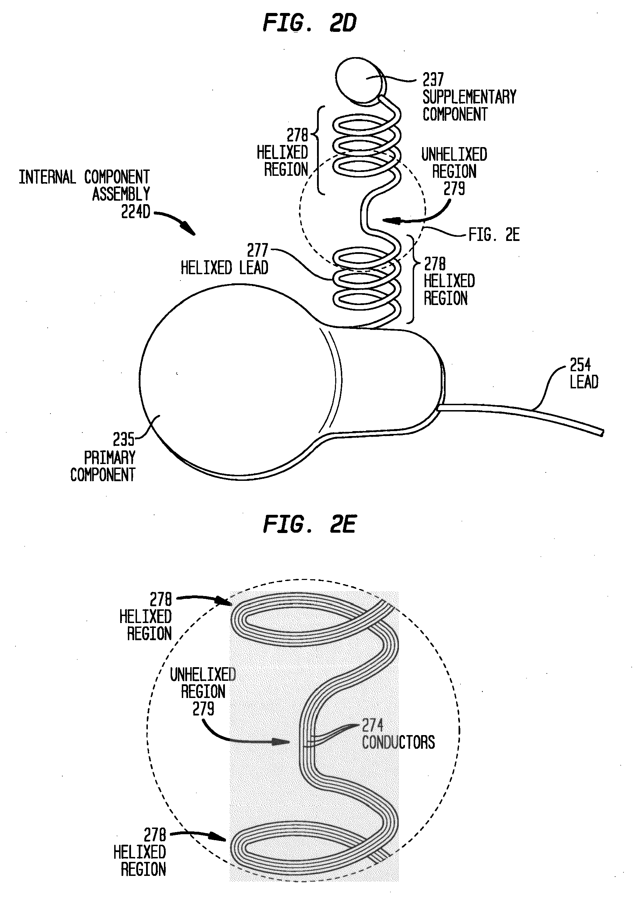

FIG. 2D is a perspective view of a portion of another exemplary internal component assembly 224D of a cochlear implant. Internal component assembly 224D is similar to internal component assembly 224C, but includes a strain relief in the form of, for example, a partly helixed lead 277 electrically connecting primary and supplementary components 235 and 237 rather than lead 276. Helixed lead 277 includes helixed regions 278 and an unhelixed region 279, which is illustrated in more detail in FIG. 2E. As will be described further below, when replacing supplementary component 237 with a replacement component unhelixed region 279 provides an advantageous location at which a lead extending from the replacement component may be coupled to lead 277 via an implantable insulated lead connector 299. As one of ordinary skill in the art would appreciate, alternative forms of strain relief are possible, such as a bundle of undulating wires.

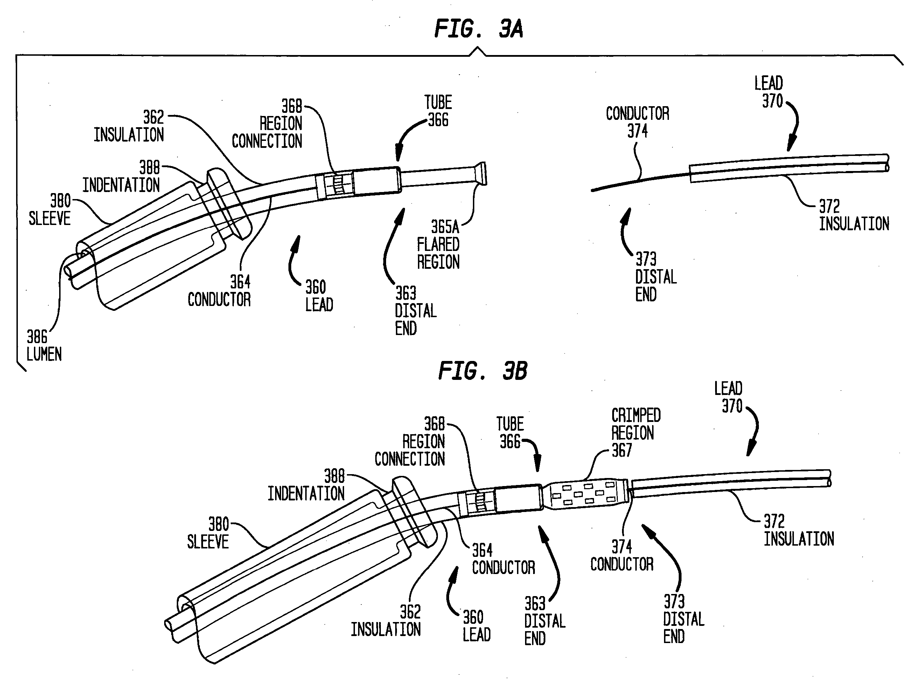

FIGS. 3A-3D are side views illustrating an exemplary process for connecting leads 360 and 370 of respective device components of an implantable medical device using an implantable electrically insulated lead connector 399. As shown in FIG. 3A, lead 370 includes a conductor 374 partially covered by substantially transparent insulation 372. In other cases, lead 370 may include more than one conductor 374. In still other cases, insulation 372 may be semi-opaque or opaque. As illustrated in FIG. 3A, insulation 372 does not cover conductor 374 at distal end 373 of lead 370. In some cases, lead 370 is a flying lead connected at a proximal end to a primary component 235 of a cochlear implant and manufactured with insulation 372 completely covering conductor 374. In such cases, insulation 372 is stripped from conductor 374 at distal end 373 prior to connecting lead 370 to lead 360. Insulation 372 may be stripped using any suitable tool or method, and may be stripped during a surgical procedure to implant the secondary implantable module, for example. In addition, lead 370 may be shortened, if desired, before it is electrically connected to lead 360. In such cases, a distal portion of lead 370 is severed, and insulation 372 is then stripped from conductor 374 at the distal end 373 remaining after shortening lead 370.

As illustrated in FIG. 3A, lead 360 includes a conductor 364 covered by transparent insulation 362. In other cases, lead 360 may include more than one conductor 364. In still other cases, insulation 362 may be semi-opaque or opaque. Lead 360 also comprises a conductor connector for electrically connecting conductors of leads 360 and 370 to thereby electrically connect leads 360 and 370. In the illustrative case of FIG. 3A, the conductor connector is a conductive tube 366 having a flared region 365A and a connection region 368 at which tube 366 is crimped to conductor 364 and thereby electrically connected to conductor 364. Insulation 362 covers a portion of tube 366, including connection region 368. In certain cases, a proximal end of lead 360 is connected to a supplementary component 237 (see FIG. 2A). In such cases, supplementary component 237 may be manufactured with tube 366 forming part of lead 360 so that lead 360 may be connected to another lead without the need for additional preparation of lead 360 (e.g., the stripping of insulation 362) prior to electrically connecting lead 360 to another lead.

In the illustrative case of FIG. 3A, a sleeve 380 is positioned around a portion of lead 360 before electrically connecting leads 360 and 370. Alternatively, sleeve 380 may be positioned around a portion of lead 370 before electrically connecting leads 360 and 370. Sleeve 380 may be a sleeve, collar or boot (collectively and generally referred to as a "sleeve"). In the illustrative case of FIGS. 3A-3D, sleeve 380 comprises a lumen 386 that extends through sleeve 380, and external indentations 388 located at both ends of sleeve 380. Sleeve 380 is configured to be longitudinally displaced (e.g., moved or slid) along leads 360 and 370, and is formed of a biocompatible material. In certain cases, sleeve 380 is formed of a non-conductive material such as silicone. In the case illustrated in FIG. 3A, sleeve 380 is substantially transparent, which may be beneficial for curing ultraviolet (UV) curable silicone disposed in sleeve 380.

FIG. 3B is a side perspective view of several components of implantable insulated lead connector 399 after electrically connecting leads 360 and 370.

Referring to FIGS. 3A and 3B, the portion of conductor 374 exposed at distal end 373 of lead 370 is inserted into tube 366 through flared region 365A to electrically connect leads 360 and 370. After the insertion of conductor 374, a portion of tube 366 (including flared region 365A) is crimped to form a crimped region 367. Once crimped region 367 is formed, conductors 364 and 374 are each electrically connected to conductive tube 366, and as such, leads 360 and 370 are electrically connected by tube 366. Tube 366 may be crimped using any suitable crimping tool, such as surgical needle holders or forceps.

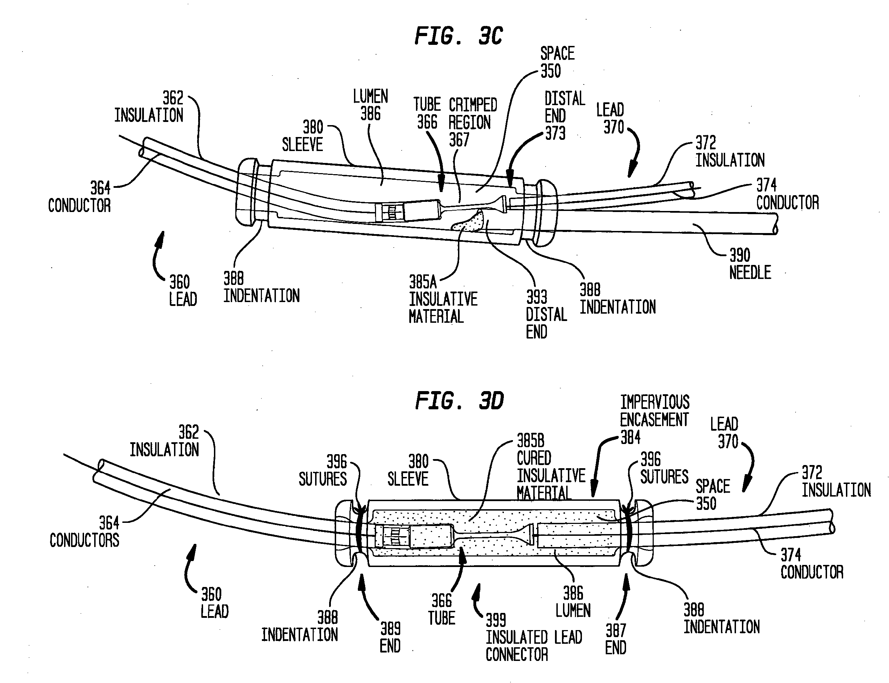

Referring to FIGS. 3B and 3C, sleeve 380 is longitudinally displaced along lead 360 and onto a portion of lead 370 until it is positioned around and encasing tube 366. As shown in FIG. 3C, the diameter of lumen 386 is large enough that, once sleeve 380 is positioned around tube 366, a space 350 is present between an inner surface of sleeve 380 and an outer surface of tube 366. In certain cases, after sliding sleeve 380 over tube 366, a distal end 393 of a needle 390 may be inserted through one end of sleeve 380 and into lumen 386. In other cases, while sleeve 380 is positioned such that it is not covering tube 366, needle 390 may be positioned such that distal end 393 is adjacent or proximal to crimped region 367. Sleeve 380 may then be longitudinally displaced along lead 360 until it is positioned around tube 366 and distal end 393 of needle 390. Space 350 disposed within sleeve 380 may then be filled with an electrically insulative material 385A via needle 390 such that insulative material 385A occupies substantially all of space 350. In certain cases, insulative material 385A is a fluent electrically insulative material that is capable of flowing from needle 390 into sleeve 380. While filling space 350, insulative material 385A conforms around tube 366 and other portions of leads 360 and 370 disposed in sleeve 380. For example, in the illustrative case of FIGS. 3A-3D, insulative material 385A will conform around tube 366 and the portion of conductor 374 that is exposed within sleeve 380 prior to filling space 350 with insulative material 385A. Electrically insulative material 385A may be a liquid, a viscous liquid or a semisoft material such as a paste or gel.

In the illustrative case of FIGS. 3A-3D, insulative material 385A is dispensed from distal end 393 of needle 390 into lumen 386 of sleeve 380 and retained in space 350 between sleeve 380 and tube 366. In certain cases, insulative material 385A is a curable electrically insulative material, such as curable silicone. For example, insulative material 385A may be a type of room-temperature vulcanizing (RTV) silicone. In certain cases, insulative material 385A may be a type of silicone curable by one or more of ultraviolet (UV) light, heat and moisture (such as moisture in the body). In some cases, insulative material 385A is curable in situ. As used herein in situ curable insulative material is electrically insulative material that is curable via exposure to conditions that will not significantly and/or adversely damage a recipient's bodily tissue when the insulative material is cured in close proximity to the bodily tissue. In certain applications, it may be necessary to cure the insulative material relatively near a recipient's bodily tissue.

After filling sleeve 380 and removing needle 390 from lumen 386, the curable insulative material 385A is cured using any suitable means in order to form an ionically impervious encasement 384 around tube 366 and to thereby form implantable insulated lead connector 399. In the illustrative case of FIG. 3D, implantable insulated lead connector 399 comprises tube 366 and an impervious encasement 384, which is disposed around tube 366. Impervious encasement 384 includes sleeve 380 and cured electrically insulative material 385B. Insulative material 385A is cured while it is conformed around tube 366 and any other exposed conductors. As such, once insulative material 385A is cured, there is no electrically conductive passageway between cured, electrically insulative material 385B and lead 360 or 370 for any substantial amount of body fluid or tissue to reach tube 366 or any other conductor covered by cured insulative material 385B. Accordingly, impervious encasement 384, which includes cured, electrically insulative material 385B, substantially prevents the ingress of electrically conductive body fluid and tissue to prevent the formation of any substantial electrically conductive path of body fluid and/or tissue from tube 366 out of impervious encasement 384, and thereby electrically insulates tube 366.

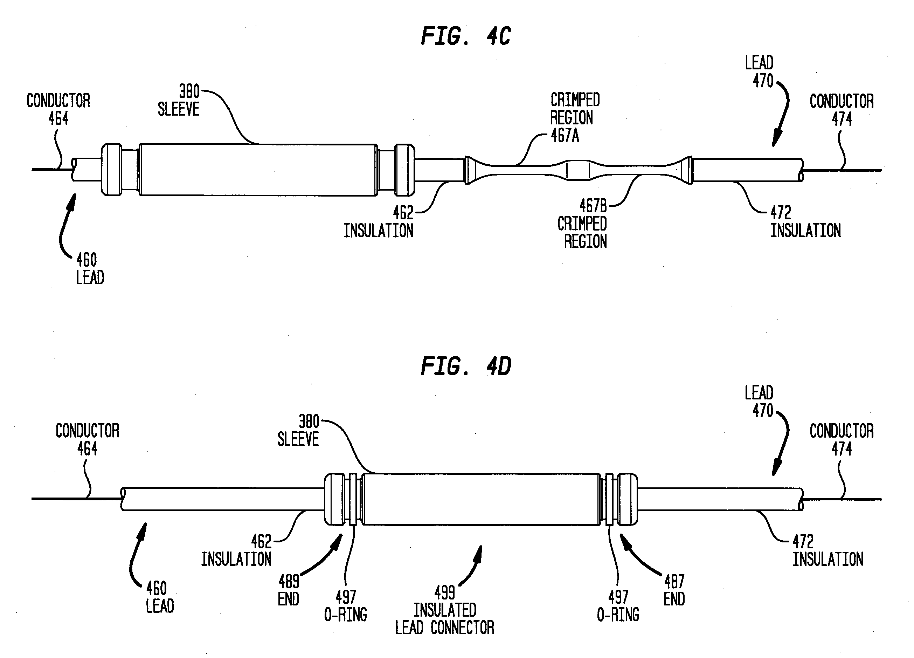

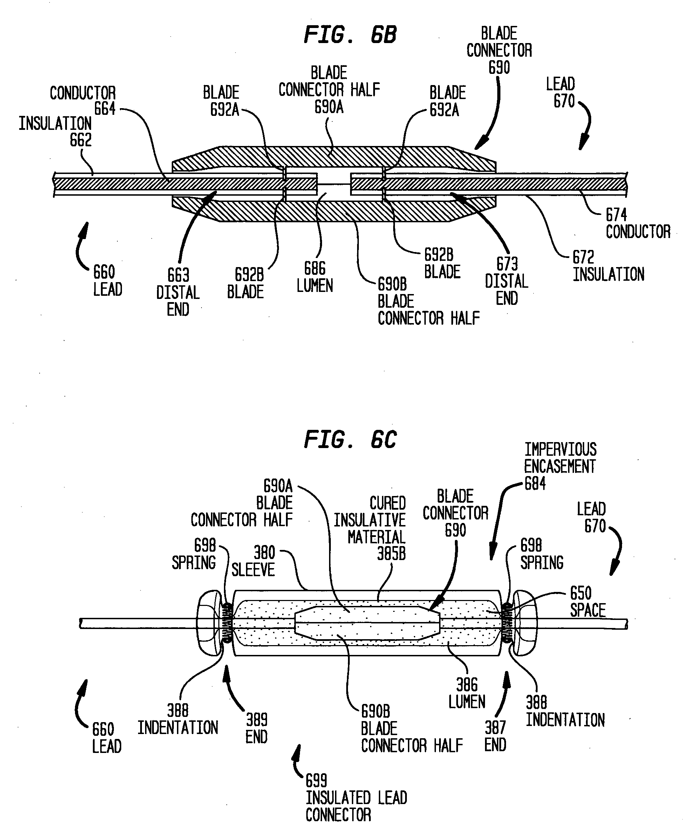

Lumen 386 may be further sealed by securing ends 387 and 389 of sleeve 380 to leads 370 and 360 via sealing elements, such as sutures, O-rings, and/or toroidal springs that will compress ends 387 and 389. Such sealing elements may be applied so that ends 387 and 389 may resist the entrance of moisture, such as body fluid, into sleeve 380. In the illustrative case of FIG. 3D, sutures 396 are applied to indentations 388 located at ends 387 and 389 of sleeve 380 to compress portions of sleeve 380 to secure sleeve 380 to leads 360 and 370. In the illustrative case of FIG. 3D, more than one suture is applied to sleeve 380 at each indentation. In other cases, sleeve 380 may be secured with more or fewer sutures 396 than the number shown in FIG. 3D, and may be secured to leads 360 and 370 with a single suture 396 in each indentation 388. Alternatively, O-rings made from silicone or rubber, for example, may be placed around indentations 388 to further seal lumen 386 (see, e.g., FIG. 4D). Also, in other cases, toroidal springs may be placed around indentations 388 to further seal lumen 386 (see, e.g., FIG. 6C). Each of the toroidal springs may have an inner diameter, in an equilibrium or unstretched state, that is smaller than the outer diameter of the lead 360 or 370 and/or the indentation 388 around which it is to be placed so that the toroidal spring compresses sleeve 380 to the lead 360 or 370 when placed around indentation 388.

In the illustrative case of FIG. 3D, sutures 396 are applied after curing insulative material 385A. In other cases, a sealing element may be provided at end 389 prior to filling lumen 386 with insulative material 385A to resist the movement of insulative material 385A out of end 389 before insulative material 385A is cured. In such cases, a sealing element may also be provided around end 387 once lumen 386 is filled with insulative material 385A to resist the movement of insulative material 385A out of lumen 386 prior to curing.

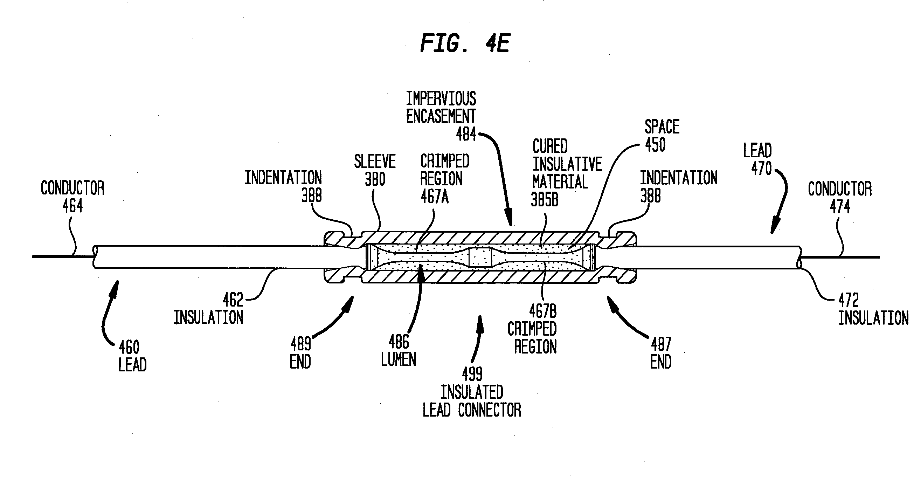

Alternatively, one or more ends of sleeve 380 may be self-sealing. As used herein, an end of a sleeve is 'self-sealing' when the end provides a seal around a lead extending through it without the assistance of any additional devices or mechanisms. An example of a self-sealing end is shown in FIG. 4E. As illustrated, the inner diameter of sleeve 480 near end 489 is small enough to provide a seal around lead 460 via a friction or interference fit.

In the illustrative case of FIG. 3D, neither of ends 387 and 389 is self-sealing. In other cases, end 389 is self-sealing while end 387 is not. In such cases, self-sealing end 389 resists the movement of insulative material 385A out of end 389 prior to curing while leading end 387 allows needle 390 to be readily inserted into lumen 386. After filling lumen 386 with insulative material 385A, end 387 may be sealed using a sealing element as described above, if desired. In other cases, both ends 387 and 389 are self-sealing. In such cases, needle 390 may be inserted under the seal of end 387, which will resume its seal around lead 370 when needle 390 is removed to resist the movement of insulative material 385A out of lumen 386. Alternatively, in some cases, when ends 387 and 389 are both self-sealing, sleeve 380 comprises a one-way valve (not shown) that allows insulative material 385A to be provided into lumen 386 but resists the movement of insulative material 385A out of the valve.

In certain cases, lead 360 is simlilar to lead 260 of FIG. 2A, lead 370 is similar to flying lead 270 of FIG. 2A, and implantable insulated lead connector 399 is similar to implantable insulated lead connector 299. In alternative cases, an implantable insulated lead connector 399 may be used to connect internal component assembly 224D of FIG. 2D with a replacement component. As illustrated in FIG. 2D, internal component assembly 224D includes a partly helixed lead 277, having helixed and unhelixed regions 278 and 279, electrically connecting primary and supplementary components 235 and 237. In certain cases, supplementary component 237 may be replaced with a replacement component having a lead 360 substantially similar to lead 360 of FIGS. 3A-3D. To replace supplementary component 237 with the replacement component, partly helixed lead 277 is severed at unhelixed region 279, with a portion of helixed lead 277 remaining connected to primary component 235. Insulation is then stripped from one or more conductors 274 at unhelixed region 279 and subsequently inserted into a tube 366 of lead 360 connected to the replacement component. Conductors 274 are then crimped within tube 366. The formation of an implantable insulated lead connector 399 may then be completed as described above in relation to FIGS. 3B-3D.

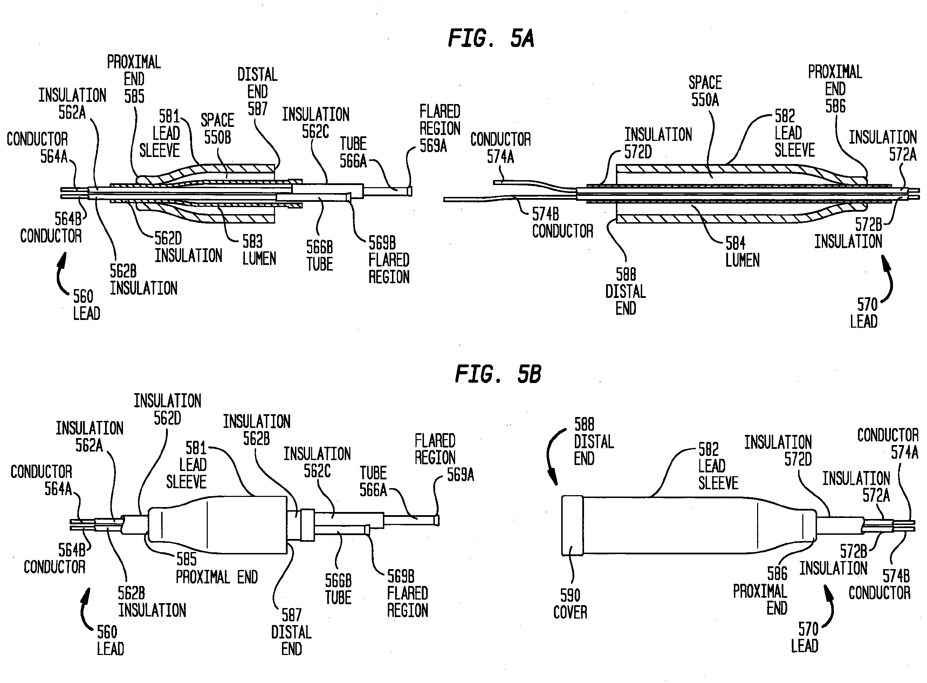

In the illustrative case of FIG. 2E, helixed lead 277 comprises multiple conductors 274. In other cases, partly helixed lead may comprise a single conductor 274. When partly helixed lead 277 comprises a single conductor 274 (e.g., a single-core conductor 274) or a plurality of electrically connected conductors 274 (e.g., a multi-core conductor 274), an implantable insulated lead conductor 399 may be used to connect partly helixed lead 277 to a lead extending from the replacement component. When partly helixed lead 277 comprises a plurality of electrically isolated conductors 274, an insulated lead conductor 599 (described below in relation to FIGS. 5A-5E) may be used to connect helixed lead 277 to a lead extending from the replacement component and also having a plurality of electrically isolated conductors. Providing an unhelixed region facilitates the severing of partly helixed lead 277, the stripping of conductor(s) 274, and the insertion of conductor(s) 274 into conductive tube(s). Additionally, when helixed lead 277 comprises a plurality of electrically isolated conductors, unhelixed region 279 provides a region in which conductors 274 may be organized to facilitate connection via an implantable insulated lead connector 599. For example, in the illustrative case of FIG. 2E, conductors 274 are arranged side-by-side in unhelixed region 279. In certain cases, this arrangement will facilitate insertion of the conductors into respective tubes 566 when connecting partly helixed lead 277 to a lead 570 of a replacement component.

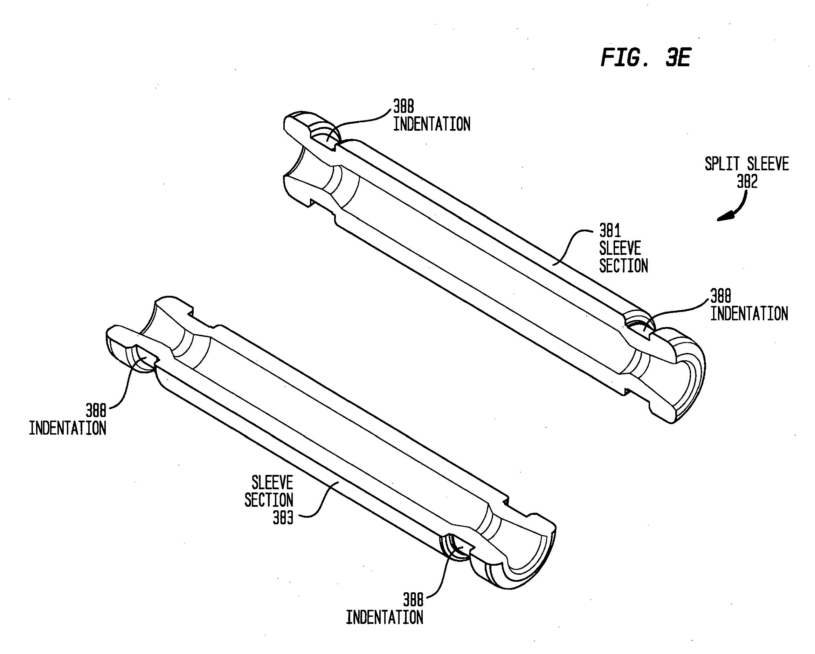

FIG. 3E is a perspective view of a longitudinally split sleeve of an implantable insulated lead connector. Longitudinally split sleeve 382 is similar to sleeve 380 shown and described in relation to FIGS. 3A-3D, except that sleeve 382 is split into two longitudinal sleeve sections 381 and 383 configured to mate to thereby form sleeve 382 having a lumen 386 (see FIG. 3C). That is, a lumen 386 is formed between sleeve sections 381 and 383 when they are mated. In some cases , sleeve 382 may be used to form an implantable insulated lead connector 399 instead of sleeve 380. In such cases, after crimping tube 366 to conductor 374 to electrically connect leads 360 and 370, longitudinal sleeve sections 381 and 383 are mated around tube 366 such that tube 366 is encased in a lumen 386. The diameter of lumen 386 is large enough that, once sleeve 382 is positioned around tube 366, a space 350 is present between an inner surface of sleeve 382 and an outer surface of tube 366. Longitudinal sleeve sections 381 and 383 may then be secured together using sealing elements, such as sutures, O-rings, and/or toroidal springs. In certain cases, the sealing elements may be applied at ends 387 and 389 of sleeve 382 (see FIG. 3C). After longitudinal sleeve sections 381 and 383 are secured together, an implantable insulated lead connector 399 may be completed as described above with reference to FIGS. 3C and 3D. A longitudinally split sleeve may also be used in other implantable insulated lead connectors. Additionally, as would be appreciated by one of ordinary skill in the art, longitudinal sleeve sections 381 and 383 may be connected by one or more hinges.

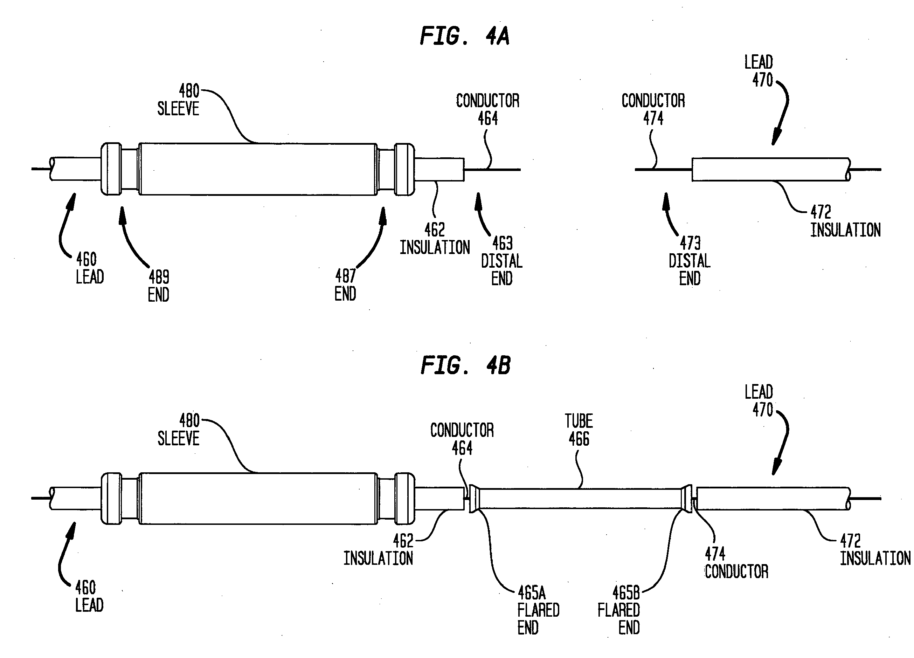

FIGS. 4A-4D are side views illustrating an exemplary process for connecting leads 460 and 470 of respective device components of an implantable medical device using an implantable electrically insulated lead connector 499. FIG. 4E is a cross-sectional view of implantable insulated lead connector 499 of FIG. 4D. In certain cases, lead 470 is a flying lead that is implanted with insulation 472 completely covering conductor 474 and lead 460 is manufactured with insulation 462 completely covering conductor 464. In the illustrative case of FIGS. 4A-4E, prior to connecting leads 460 and 470, insulation 462 is stripped from conductor 464 at distal end 463 and insulation 472 is stripped from conductor 474 at distal end 473.

As illustrated in FIG. 4A, a sleeve 480 is positioned around a portion of lead 460. Sleeve 480 is similar to sleeve 380, except that sleeve 480 is opaque and is self-sealing at ends 487 and 489. In alternative cases, sleeve 480 may be substantially transparent, and one or both of ends 487 and 489 may not be self-sealing. Referring to FIGS. 4A and 4B, once the insulation has been stripped from conductors 464 and 474, the exposed portions of conductors 464 and 474 may be inserted into a conductor connector. In the illustrative case of FIGS. 4A-4E, the conductor connector is a conductive tube 466 comprising flared ends 465A and 465B. The exposed portions of conductors 464 and 474 are be inserted into flared ends 465A and 465B of tube 466, respectively, as illustrated in FIG. 4B.

Referring to FIG. 4C, a portion of tube 466 may be crimped to conductor 464 to form a crimped region 467A and another portion of tube 466 may be crimped to conductor 474 to form a crimped region 467B. Once conductive tube 466 is crimped to conductors 464 and 474, conductors 464 and 474 are electrically connected via conductive tube 466. Sleeve 480 is then longitudinally displaced along lead 460 until it is positioned around and encasing tube 466. As shown in FIG. 4E, the diameter of a lumen 486 of sleeve 480 is large enough that, once sleeve 480 is positioned around tube 466, a space 450 is present an inner surface of sleeve 480 and an outer surface of tube 466. Space 450 disposed within sleeve 480 is then filled with an electrically insulative material, such as one of the electrically insulative materials described above in relation to FIGS. 3A-3D. While filling space 450, the electrically insulative material conforms around tube 466 and other portions of leads 460 and 470 disposed in sleeve 380. As described above, the insulative material may be a curable electrically insulative material. FIG. 4E is a cross-sectional view of implantable insulated lead connector 499 of FIG. 4D. Referring to FIG. 4E, when a curable insulative material is used, the curable insulative material is then cured to form an ionically impervious encasement 484 around tube 466 to thereby form implantable insulated lead connector 499. Implantable insulated lead connector 499 comprises tube 466 and impervious encasement 484. Impervious encasement 484 includes sleeve 480 and cured electrically insulative material 385B. Impervious encasement 484 is similar to impervious encasement 384 described above in relation to FIGS. 3A-3E.

After curing the electrically insulative material, ends 487 and 489 of sleeve 480 may be secured to leads 460 and 470 via sealing elements such as sutures, O-rings, and/or torodial springs, as described above in relation to FIGS. 3A-3E. Application of the sealing elements may further seal lumen 486. In the illustrative case of FIG. 4D, O-rings 497 are applied to sleeve 480 at indentations 488. As described above, ends 487 and 489 of sleeve 480 are self-sealing. However, one or more sealing elements may additionally be applied to sleeve 480 to further resist the movement of electrically conductive material from the implanted environment (e.g., electrically conductive bodily fluid and tissue) into sleeve 480.

As illustrated in FIG. 4E, ends 487 and 489 of sleeve 480 are self-sealing and form a friction or interference fit with leads 470 and 460, respectively. As illustrated, an inner diameter of sleeve 480 at end 489 is smaller than an outer diameter of lead 460 so that end 489 will form an interference fit with lead 460. Similarly, an inner diameter of sleeve 480 at end 487 is smaller than an outer diameter of lead 470 so that end 487 will form an interference fit with lead 470. Cured electrically insulative material 385B is illustrated schematically via small dots in FIG. 4E. Cured electrically insulative material 385B substantially fills space 450, is conformed to tube 466 and substantially prevents the ingress of electrically conductive body fluid and tissue.

Implantable insulated lead connector 499 may be used to replace supplementary component 237 of an implanted internal component assembly 224C. In an exemplary case, after surgically accessing supplementary component 237 and lead 276, lead 276 may be severed and supplementary component 237 may be explanted. Subsequently, a new supplementary component may be implanted and a lead extending from the new supplementary component may be connected to the portion of lead 276 connected to primary component 235 substantially as described above in relation to FIGS. 4A-4E. Alternatively, in certain cases, the new supplementary component includes a lead similar to lead 360, and the new supplementary component may be connected to the portion of lead 276 connected to primary component 235 substantially as described above in relation to FIGS. 3A-3D.

In the cases described above in relation to FIGS. 4A-4E, conductors 464 and 474 are inserted into a conductive tube 466 to electrically connect leads 460 and 470. In alternative cases, a conductive pin may be attached to one or more of conductors 464 and 474 to facilitate the electrical connection of leads 460 and 470. For example, in certain cases after stripping insulation from distal ends 463 and 473 of leads 460 and 470, a conductive pin is attached to each of the exposed conductors 464 and 474. The pins may be attached to the conductors using any suitable method, such as using adhesiyes, welding or crimping. Once attached, the pins are inserted into flared ends 465A and tube 466 is then crimped around the pins. In other cases, a pin is attached to conductor(s) of only one of leads 460 and 470. Additionally, in some cases, a lead may be manufactured with a conductive pin extending from the distal end of the lead. For example, in cases in which implantable insulated lead connector 499 is used to connect a primary component to a supplementary component, as described above in relation to FIGS. 2A and 2B, lead 460 of the supplementary component may be manufactured with a conductive pin electrically connected to conductor(s) 464 and disposed at distal end 463 to simplify the electrical connection of lead 460 and 470. In such cases, the pin may be inserted into tube 466, and as such, lead 460 may be electrically connected to tube 466 without the need to first strip insulation 462 from conductor(s) 464. Additionally, when lead 460 is manufactured with a pin at the distal end, the pin may be partially encapsulated to further secure the pin to the lead and to facilitate handling of the pin.

An advantage of the cases described above in relation to FIGS. 4A-4E is that an implantable insulated lead connector 499 may be used to create an encapsulated electrical connection between any two leads and at nearly any location along either of the leads. Implantable insulated lead connector 499 does not require leads manufactured with any particular connectors and may even be used to connect leads with incompatible connectors by first severing the incompatible connectors from the distal ends of the leads. Implantable insulated lead connector 499 may also be used to repair a lead extending between device components.

Referring to FIG. 2C, for example, implantable insulated lead connector 499 may be used to repair lead 276, which electrically connects primary and supplementary components 235 and 237. Lead 276 may require repair when, for example, one or more conductors of lead 276 becomes exposed or a break in lead 276 occurs. When a complete break has occurred in lead 276, the portion of lead 276 connected to primary component 235 and the portion of lead 276 connected to supplementary component 237 may be connected via an implantable insulated lead connector 499, as described above in relation to FIGS. 4A-4E. Alternatively, if a fault other than a complete break has occurred, then lead 276 may be cut at the site of the fault or a portion of lead 276 containing the fault may be removed. Thereafter, the portion of lead 276 connected to primary component 235 and the portion of lead 276 connected to supplementary component 237 may be connected via an implantable insulated lead connector 499, as described above in relation to FIGS. 4A-4E. Repairing lead 276 is simpler and less surgically invasive than explanting and replacing internal component assembly 224C when a lead of internal component assembly 224C has failed.

Additionally, implantable insulated lead connector 499 may be used to customize the length of a lead of an implantable medical device. To shorten a lead, for example, a section of the lead may be removed and the remaining portions of the lead may be reconnected using an implantable insulated lead connector 499. To lengthen a lead, the lead may be severed and then an additional lead section may be connected between the severed portions of the original lead via two implantable insulated lead connectors 499. As such, a daisy chain of leads may be created using implantable insulated lead connectors 499 to link the leads together. Similar advantages may be provided by other cases described herein in which the leads are first stripped of insulation before being electrically connected.

FIG. 5A is a cross-sectional view of portions of an implantable electrically insulated lead connector 599 configured to form multiple electrical connections between leads of respective device components of an implantable medical device. FIGS. 5B-5E are side views illustrating an exemplary process for connecting leads 560 and 570 of respective device components of an implantable medical device using an implantable insulated lead connector 599.

An exemplary process for connecting multi-conductor leads 560 and 570 via implantable insulated lead connector 599 is described below with reference to FIGS. 5A-5D. In the illustrative case of FIG. 5A, lead 570 includes conductors 574A and 574B, which are partially covered by insulation 572D. Conductor 574A is partially covered by insulation 572A and conductor 574B is partially covered by insulation 572B and thus conductors 574A and 574B are electrically isolated from one another. As such, in certain cases, implantable insulated lead connector 599 is capable of connecting multipolar leads 560 and 570. In some cases, each of conductors 574A and 574B is a plurality of conductors. In other cases, lead 570 includes three or more conductors electrically isolated from one another. In the illustrative case of FIG. 5A, lead 570 also includes a lead sleeve 582 having a lumen 583 and an open distal end 588. Lead sleeve 582 may be longitudinally displaced along lead 570.

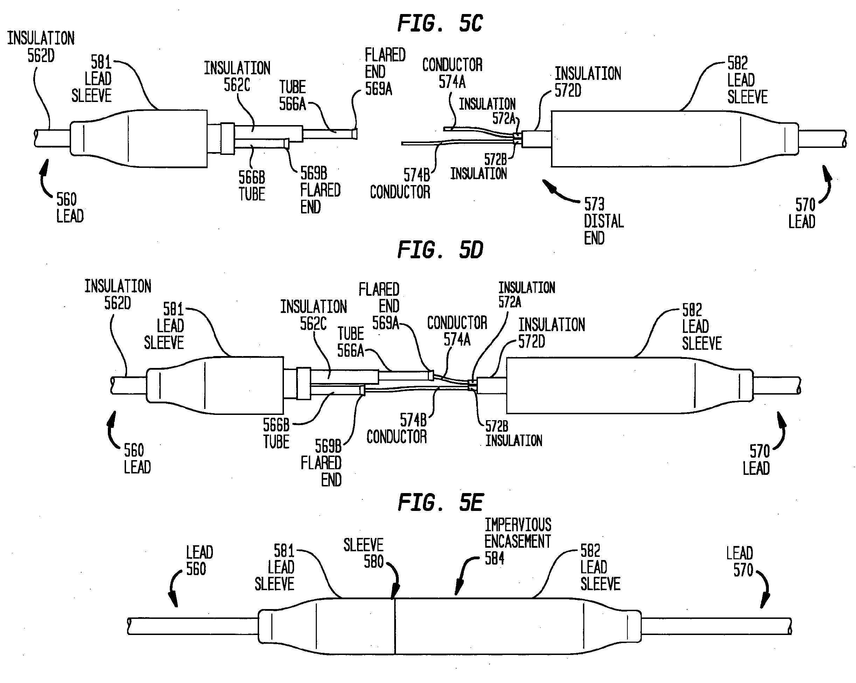

In the illustrative case of FIGS. 5A-5E, lead 570 is a flying lead in which conductors 574A and 574B are electrically isolated from the surrounding environment when lead 570 is initially implanted. As shown in FIG. 5B, for example, lead 570 may be initially implanted with lead sleeve 582 covering conductors 574A and 574B, with distal end 588 covered by a removable cover 590, such as a removable lid or layer of insulation. Referring to FIGS. 5B and 5C, before electrically connecting leads 560 and 570, removable cover 590 is removed and lead sleeve 582 is longitudinally displaced away from distal end 573 along lead 570 to expose portions of conductors 574A and 574B. Alternatively, lead 570 may be implanted with the portions of conductors 574A and 574B disposed at distal end 573 positioned in any suitable insulating sheath, cover or package (collectively and generally referred to as a "package"). The package is removed prior to electrically connecting leads 560 and 570.

Lead 560 is similar to lead 360 illustrated in FIGS. 3A-3D, except that lead 560 includes multiple conductors electrically isolated from one another by insulation 562A and 562B, and includes a conductor connector electrically connected to each of those conductors. As illustrated in FIG. 5A, lead 560 includes conductors 564A and 564B, which are partially covered by insulation 562D. Conductor 564A is partially covered by insulation 562A and conductor 564B is partially covered by insulation 562B and the conductors 564A and 564B are electrically isolated from one another. Like lead 570, in alternative cases, lead 560 may include three or more conductors that are isolated from one another. In certain cases, each of the electrically isolated conductors corresponds to a plurality of conductors.

Lead 560 includes a plurality of conductor connectors respectively connected to the electrically isolated conductors of lead 560. In the illustrative case of FIG. 5A, the conductor connectors are tubes 566A and 566B. Conductor 564A is electrically connected to conductive tube 566A having a flared distal end and conductor 564B is electrically connected to conductive tube 566B having a flared distal end. Tubes 566A and 566B may be crimped to conductors 564A and 564B, respectively, and are electrically isolated from one another by insulation 562C, which covers a portion of tube 566A. In the illustrative case of FIG. 5A, the flared ends of tubes 566A and 566B are offset from one another. Alternatively, the ends of tubes 566A and 566B may be even with one another.

Lead 560 also includes a lead sleeve 581 having a lumen 583 and an open distal end 587. Distal end 587 of lead sleeve 581 is configured to mate with distal end 588 of lead sleeve 582, and a seal may be formed where distal ends 587 and 588 mate. In the illustrative case of FIG. 5A, distal ends 587 and 588 have substantially the same diameter. In certain cases, lead sleeve 581 is secured to insulation 562D of lead 560, and may not be longitudinally displaced along lead 560. In other cases, lead sleeve 581 is not secured to lead 560 may be longitudinally displaced along lead 560. Each of lead sleeves 581 and 582 is formed of a biocompatible, non-conductive material, such as silicone. In the illustrative case of FIGS. 5A-5E, lead sleeve 581 is shorter than lead sleeve 582. In other cases, lead sleeve 581 may be longer than lead sleeve 582 or they may have equal lengths. In certain cases, one or both of lead sleeves 581 and 582 are capable of being longitudinally displaced along leads 560 and 570, respectively.

As illustrated in FIGS. 5A and 5C, insulation 572D does not cover conductors 574A and 574B at distal end 573 of lead 570. As illustrated in FIG. 5D, the exposed ends of conductors 574A and 574B are inserted into tubes 566A and 566B, respectively. More specifically, conductor 574A is inserted into flared end 569A of tube 566A and conductor 574B is inserted into flared end 569B of tube 566B. Tubes 566A and 566B are crimped to secure conductors 574A and 574B within tubes 566A and 566B, respectively, thereby completing the electrical connection of leads 560 and 570. Specifically, after crimping the conductors within the tubes as described above, conductor 574A is electrically connected to conductor 564A via conductive tube 566A and conductor 574B is electrically connected to conductor 564B via conductive tube 566B. Tubes 566A and 566B may be crimped using any suitable crimping tool, such as surgical needle holders or forceps, as described above with regard to FIGS. 3A-3D.

Referring to FIG. 5E, after crimping the conductors within the tubes as described above, lead sleeve 582 is longitudinally displaced along lead 570 toward lead sleeve 581 to mate distal end 588 of lead sleeve 582 with distal end 587 of lead sleeve 581 to form a laterally split sleeve 580 around and encasing tubes 566A and 566B. In alternative cases, lead sleeve 581 may be longitudinally displaced along lead 560 toward lead sleeve 582 to mate lead sleeves 581 and 582, or both lead sleeves 581 and 582 may be longitudinally displaced toward one another to mate.

In certain cases, sleeve 580 is sealed where distal ends 587 and 588 mate. For example, distal ends 587 and 588 may be bonded together. In one case, the above bonding is performed by disposing a glue layer on one or more of distal ends 587 and 588 and pressing together distal ends 587 and 588. Alternatively, a liquid glue may be applied between distal ends 587 and 588. In one case, the liquid glue sets and/or cures rapidly. In another case, a UV-curable glue is pre-applied to one or more of distal ends 587 and 588, or is applied as a liquid, or is a separate component that is inserted between distal ends 587 and 588. In one case, a liquid perfluoropolymer such as that described in International Application WO 2007/021620 A2 may be utilized. Other adhesives include, but are not limited to, fibrin glues, cyanoacrylates, polyurethane adhesives, silicone adhesives and UC-curable acrylics. In another case, chemical surface modification may be utilized to attain a desired bonding.

After mating lead sleeves 581 and 582 to form sleeve 580, tubes 566A and 566B are positioned in a lumen 583, 584 of sleeve 580. Lumen 583, 584 is large enough that a space 550A, 550B is present between an inner surface of sleeve 580 and outer surfaces of tubes 566A and 566B. With lead sleeves 581 and 582 mated, space 550A, 550B within sleeve 580 is then filled with electrically insulative material as described above in relation to FIGS. 3A-3D. While filling space 550A, 550B, the electrically insulative material conforms around each of tubes 556A and 566B and other portions of leads 560 and 570 disposed in sleeve 580. As described above, the electrically insulative material may be a curable electrically insulative material. When a curable electrically insulative material is used, the curable insulative material is then cured using any suitable means to form an ionically impervious encasement 584 around tubes 566A and 566B to thereby form an implantable insulated lead connector 599. Implantable insulated lead connector 599 comprises tubes 566A and 566B, and impervious encasement 584. Impervious encasement 584 includes sleeve 580 and cured electrically insulative material 385B (see, e.g., FIG. 4E) filling space 550A, 550B. As described above in relation to impervious encasement 384, impervious encasement 584 substantially prevents the ingress of electrically conductive body fluid and tissue to prevent the formation of any substantial conductive path of body fluid and/or tissue from tubes 566A and 566B out of impervious encasement 584 and thereby electrically insulates tubes 566A and 566B.

In some cases, the electrically insulative material is an in situ curable electrically insulative material, as described above. In certain cases, one or more of proximal ends 585 and 586 of lead sleeves 581 and 582 is self-sealing. Additionally or alternatively, proximal ends 585 and 586 may be secured to leads 560 and 570 to further seal lumen 583, 584 using sealing elements such as sutures, O-rings, and/or toroidal springs, as described above.

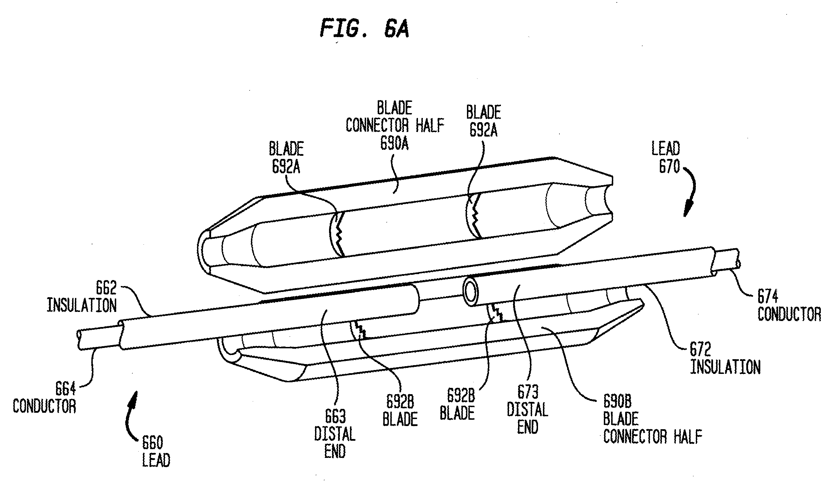

FIGS. 6A-6C illustrate an exemplary process for connecting leads 660 and 670 of respective device components of an implantable medical device using an implantable electrically insulated lead connector 699. FIG. 6A is a perspective view of components of an implantable insulated lead connector 699.

An exemplary process for connecting leads 660 and 670 via an implantable insulated lead connector 699 will be described below with reference to FIGS. 6A-6C. In the illustrative case of FIG. 6A, lead 670 includes a conductor 674 covered by insulation 672. In other cases, lead 670 may include more than one conductor 674. In certain cases, lead 670 is implanted with insulation 672 completely covering conductor 674. Lead 660 includes a conductor 664 covered by insulation 662 and, in some cases, insulation 662 completely covers conductor 674. In certain cases, lead 660 may include more than one conductor 664.

Unlike the cases described in relation to FIGS. 3 and 4, in the illustrative case of FIGS. 6A-6C, leads 660 and 670 may be electrically connected without stripping insulation 662 and 672 at distal ends 663 and 673 of leads 660 and 670. Rather, leads 660 and 670 are electrically connected via an insulation displacement connection (IDC). In the illustrative case of FIG. 6A, the conductor connector that electrically connects leads 660 and 670 is a blade connector 690 (see FIG. 6A) comprising blade connector halves 690A and 690B. Blade connector half 690A includes a plurality of blades, spikes or teeth, (collectively and generally referred to as "blades") 692A capable of penetrating insulation 672 and 662 to make contact with conductors 674 and 664. Similarly, blade connector half 690B includes a plurality of blades 692B capable of penetrating insulation 672 and 662 to make contact with conductors 674 and 664.

FIG. 6B is a cross-sectional view of components of an implantable insulated lead connector 699. Referring to FIG. 6B, blade connector halves 690A and 690B are mated such that blade connector 690 encloses distal ends 663 and 673 of leads 660 and 670, respectively, within a lumen 686 extending through blade connector 690. When blade connector 690 is mated around leads 660 and 670, as illustrated in FIG. 6B, blades 692A and 692B pierce (or otherwise displace) insulation 662 and 672 and make contact with conductors 664 and 674. In certain cases, blades 692A and 692B are sharp enough to penetrate insulation 692A and 692B when blade connector 690 is squeezed by hand to mate blade connector halves 690A and 690B around distal ends 663 and 673.

In the illustrative case of FIGS. 6A-6C, blades 692A and 692B are conductive, as are blade connector halves 690A and 690B. As such, when blades 692A and 692B make contact with conductors 664 and 674, as shown in FIG. 6B, conductors 664 and 674 are electrically connected via blades 692A and the conductive body of blade connector half 690A, and via blades 692B and the conductive body of blade connector half 690B. In the illustrative case of FIG. 6B, blades 692A and blade connector half 690A are unitary, and blades 692B and blade connector half 690B are unitary. Alternatively, blades 692A may be formed separately from blade connector half 690A and subsequently physically and electrically connected to blade connector half 690A and blades 692B may be formed separately from blade connector half 690B and subsequently physically and electrically connected to blade connector half 690B. In other cases, instead of blade connector 690, implantable insulated lead connector 699 may include an insulation displacement connector having at least two conductive screws. In such cases, two halves of the insulation displacement connector may be mated such that they enclose distal ends 663 and 673 like blade connector 690. Once mated, the at least two screws may be operated such that one screw penetrates distal end 663 to contact conductor 664 and the other screw penetrates distal end 673 to contact conductor 674, to thereby electrically connect leads 660 and 670.

FIG. 6C is a side view of an implantable insulated lead connector 699. Referring to FIGS. 6B and 6C, after mating blade connector halves 690A and 690B around leads 660 and 670 to electrically connect leads 660 and 670, a sleeve 380 (as described above in relation to FIGS. 3A-3D) is positioned around and encasing blade connector 690. As shown in FIG. 6C, the diameter of lumen 386 is large enough that, once sleeve 380 is positioned around blade connector 690, a space 650 is present between an inner surface of sleeve 380 and an outer surface of blade connector 690. Space 650 disposed within sleeve 380 is then filled with an electrically insulative material, such as one of the electrically insulative materials described above in relation to FIGS. 3A-3D. While filling space 650, the insulative material conforms around blade connector 690 and other portions of leads 660 and 670 disposed in sleeve 380. As described above, the electrically insulative material may be a electrically curable insulative material. When a curable electrically insulative material is used, after filling space 650 with the curable electrically insulative material, the curable insulative material is cured using any suitable means in order to form an ionically impervious encasement 684 and to thereby form an implantable insulated lead connector 699. In the illustrative case of FIG. 6C, implantable insulated lead connector 699, comprises blade connector 690 and impervious encasement 684, which is disposed around blade connector 690. Impervious encasement 684 includes sleeve 380 and cured electrically insulative material 385B. As described above in relation to impervious encasement 384, impervious encasement 684 substantially prevents the ingress of electrically conductive body fluid and tissue to prevent the formation of any substantial electrically conductive path of body fluid and/or tissue from blade connector 690 out of impervious encasement 684, and thereby insulates blade connector 690.

Additionally, in certain cases, lumen 386 may be further sealed by securing ends 387 and 389 of sleeve 380 to leads 670 and 660 via sealing elements, such as sutures, O-rings, and/or toroidal springs, as described above. In the illustrative case of FIG. 6C, toroidal springs 698 are applied to sleeve 380. Each of toroidal springs 698 has an inner diameter, in an equilibrium or unstretched state, that is smaller than the outer diameter of the lead 660 or 670 and/or indentations 388. In use, each of toroidal springs 698 is stretched to expand its inner diameter, positioned over one of indentations 388, and subsequently released so that the toroidal spring compresses sleeve 388 around lead 660 or 670 as it constricts toward its equilibrium or unstretched state. In alternative cases, other types of encasing elements my be used instead of sleeve 380. Implantable insulated lead connector 699 may use insulation displacement connectors other than those described above in relation to FIGS. 6A-6C.

Implantable insulated lead connector 699 may be advantageously used to repair a lead extending between device components of an implantable medical device. For example, referring to FIG. 2B, implantable insulated lead connector 699 may be used to repair lead 276, which electrically connects primary and supplementary components 235 and 237. When a complete break has occurred in lead 276, for example, blade connector 690 may be used to connect the two separate halves of lead 276 and then insulated via an impervious encasement 684, as shown and described above in relation to FIGS. 6A-6C.

Additionally, if a fault other than a complete break has occurred, then blade connector 690 may be used to bypass the faulty portion of lead 267 by enclosing the fault with blade connector 690 such that a first pair of blades 692A and 692B is disposed on one side of the fault and a second pair of blades 692A and 692B is disposed on the other side of the fault. Blade connector 690 may then be insulated via an impervious encasement 684, as shown and described above in relation to FIGS. 6B-6C. Alternatively, lead 276 may first be cut at the site of the fault, and thereafter blade connector 690 may be used to connect the two separate halves of lead 276. Blade connector 690 may then be insulated via an impervious encasement 684, as shown an described above in relation to FIGS. 6A-6C. In addition, as described in relation to implantable insulated lead connector 499, implantable insulated lead connector 699 may be used to create an electrical connection between any two leads, and at nearly any location along either of the leads, and may be also used to customize the length of a lead of an implantable medical device.

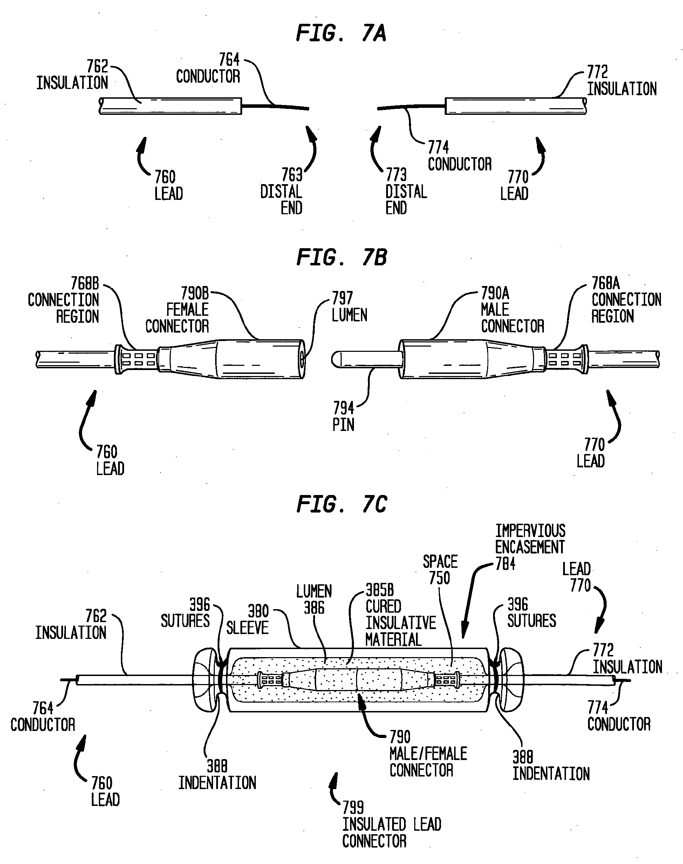

FIGS. 7A-7C are side views illustrating an exemplary process for connecting leads 760 and 770 of respective device components of an implantable medical device using an implantable electrically insulated lead connector 799. An exemplary process for connecting leads 760 and 770 via an implantable insulated lead connector 799 will be described below with reference to FIGS. 7A-7C. Lead 760 comprises a conductor 764 partially covered by insulation 762, and lead 770 comprises a conductor 774 partially covered by insulation 772. In certain cases, lead 770 is a flying lead that is implanted with insulation 772 completely covering conductor 774. As shown in FIG. 7A, insulation 772 is stripped from conductor 774 at distal end 773 prior to connecting lead 770 to lead 760. In certain cases, lead 760 is manufactured with insulation 762 completely covering conductor 764. In such cases, insulation 762 is stripped from conductor 764 at distal end 763 prior to connecting lead 760 to lead 770. Alternatively, lead 760 may be manufactured with conductor 764 exposed, or connected to a pin as described above, in order to facilitate electrically connecting lead 760 to another lead.

Referring to FIGS. 7A and 7B, once the insulation has been stripped from conductors 764 and 774 at distal ends 763 and 773, respectively, the exposed portions of conductors 764 and 774 may each be connected to a portion of a conductor connector. In the illustrative case of FIG. 7B, conductor 774 is secured and electrically connected to a male connector 790A having a conductive pin 794 and conductor 764 is secured and electrically connected to a female connector 790B having a lumen 797 configured to receive pin 794. Male and female connectors 790A and 790B are electrically connected to one another by inserting pin 794 into lumen 797. Together, male and female connectors 790A and 790B form a conductor connector referred to herein as male/female connector 790. Male and female connectors 790A and 790B may be secured and electrically connected to conductors 774 and 764, respectively, in any suitable manner. In the illustrative case of FIG. 7B, a conductive connection region 768A of male connector 790A is crimped to conductor 774 and thereby secured and electrically connected to conductor 774, and a conductive connection region 768B of female connector 790B is crimped to conductor 764 and thereby secured and electrically connected to conductor 764. In other cases, lead 760 is manufactured with female connector 790B disposed at distal end 763 and electrically connected to conductor 764 to simplify the process for connecting lead 760 to lead 770.

Referring to FIGS. 7B and 7C, male and female connectors 790A and 790B are electrically connected by inserting pin 794 into lumen 797 to thereby electrically couple leads 760 and 770. A sleeve 380, as described above, is then longitudinally displaced along lead 760 or 770 until positioned around and encasing male/female connector 790, as illustrated in FIG. 7C. In certain cases, sleeve 380 will also cover portions of leads 760 and 770 extending from male and female connectors 790A and 790B. As shown in FIG. 7C, the diameter of lumen - 386 is large enough that, once sleeve 380 is positioned around male/female connector 790, a space 750 is present between an inner surface of sleeve 380 and an outer surface of male/female connector 790. Space 750 disposed within sleeve 380 is then filled with an electrically insulative material, such as one of the electrically insulative materials described above in relation to FIGS. 3A-3D. While filling space 750, the electrically insulative material conforms around male/female connector 790 and other portions of leads 760 and 770 disposed in sleeve 380. As described above, the insulative material may be a curable electrically insulative material. When a curable insulative material is used, after filling space 750 with the curable insulative material, the curable insulative material is cured using any suitable means in order to form an ionically impervious encasement 784 and to thereby form an implantable electrically insulated lead connector 799. In the illustrative cases of FIG. 7C, implantable insulated lead connector 799, comprises male/female connector 790 and impervious encasement 784, which is disposed around male/female connector 790. Impervious encasement 784 includes sleeve 380 and cured electrically insulative material 385B. As described above in relation to impervious encasement 384, impervious encasement 784 substantially prevents the ingress of electrically conductive body fluid and tissue to prevent the formation of any substantial conductive path of body fluid and/or tissue from male/female connector 790 out of impervious encasement 784 and thereby electrically insulates male/female connector 790.

In certain cases, sealing elements may be applied to sleeve 380, as described above. In the illustrative case FIG. 7C, sleeve 380 is filled with a curable electrically insulative material that is subsequently cured and then secured with sutures 396 to form implantable electrically insulated lead connector 799. As illustrated in FIG. 7C, sutures 396 compress sleeve 380 to leads 760 and 770 to secure sleeve 380 to leads 760 and 770. Alternatively, other sealing elements, such as O-rings and/or toroidal springs, may be used to secure sleeve 380 to leads 760 and 770 and/or to further seal lumen 386. In other cases, implantable insulated lead connector 799 is formed without sealing elements.

Implantable insulated lead connector 799 may be used to repair a lead extending between device components of an implantable medical device. For example, when a complete break has occurred in the lead, then the separated portions of lead may be connected using an implantable insulated lead connector 799 as described above for connecting leads 770 and 760. Distal ends of the separate portions of the lead may be stripped, if necessary, as described above in relation to leads 770 and 760. Alternatively, if a fault other than a complete break has occurred, then lead may be cut at the site of the fault, and thereafter the two portions of the lead may be connected as described above for leads 770 and 760. In addition, as described in relation to implantable insulated lead connector 499, implantable insulated lead connector 799 may be used to create an electrical connection between any two leads, and at substantially any location along either of the leads, and may be also used to customize the length of a lead of an implantable medical device.

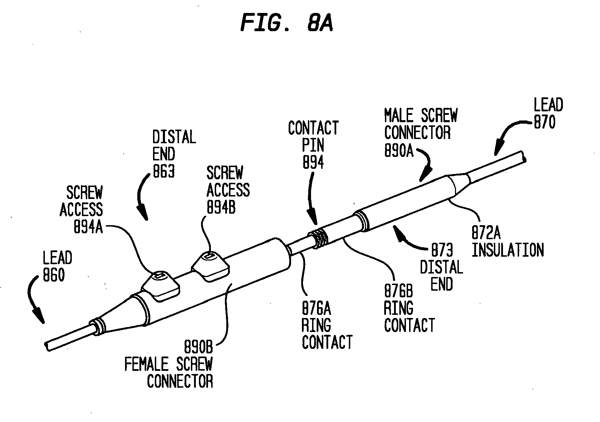

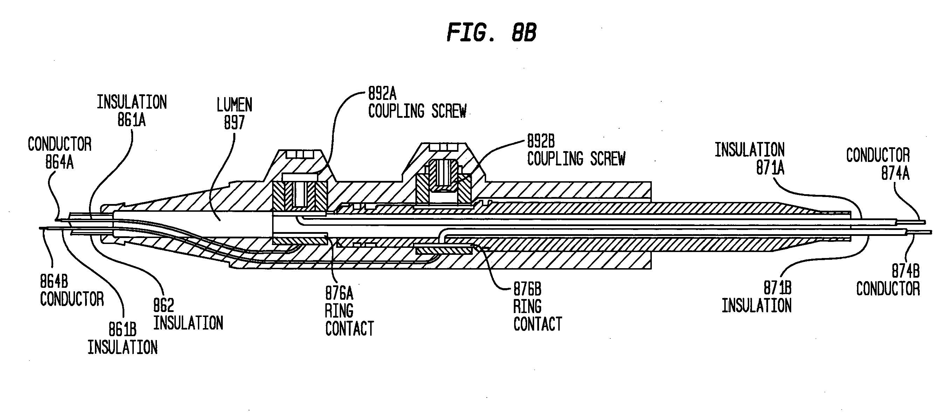

FIGS. 8A-8C illustrate an exemplary process for connecting leads 860 and 870 of respective device components of an implantable medical device using an implantable electrically insulated lead connector 899. In the illustrative case FIGS. 8A-8C, the conductor connector that electrically couples leads 860 and 870 is a screw connector (or grub screw connector) 890 comprising male and female screw connectors 890A and 890B. Referring to FIGS. 8A and 8B, lead 870 comprises conductors 874A and 874B substantially covered by insulation 872A. In the illustrative case FIGS. 8A-8C, conductors 874A and 874B are surrounded by electrical insulation 871 A and 871B such that they are electrically isolated from one another. In certain cases, lead 870 is manufactured and initially implanted with male screw connector 890A disposed at a distal end 873 of lead 870. Male screw connector 890A comprises a contact pin 894 having a diameter that tapers in a substantially stepwise manner. Contact pin 894 includes a ring contact 876A having a relatively small diameter and a ring contact 876B which has a larger diameter than ring contact 876A. Ring contacts 876A and 876B are electrically connected to conductors 874A and 874B, respectively. In certain cases, male screw connector 890A is similar to an IS-1 connector used for pacemaker leads.

Lead 860 comprises conductors 864A and 864B covered by insulation 861A and 861B, respectively, such that conductors 864A and 864B are electrically isolated from one another. In certain cases, lead 860 is manufactured with female screw connector 890B disposed at a distal end 863 of lead 860. Female screw connector 890B comprises a lumen 897 configured to receive contact pin 894. The inner diameter of lumen 897 tapers in a substantially stepwise manner such that it may receive contact pin 894. Female screw connector 890B also comprises coupling screws 892A and 892B, which are electrically connected to conductors 864A and 864B, respectively.

An exemplary process for coupling leads 860 and 870 via an implantable insulated lead connector 899 will be described below with reference to FIGS. 8A-8C. In certain cases, lead 870 may be a flying lead that is initially implanted with conductor contacts 876A and 876B covered by any suitable insulating package (not shown). The package is removed prior to electrically connecting leads 860 and 870.

Referring to FIGS. 8A and 8B, male and female screw connectors 890A and 890B are electrically connected by inserting contact pin 894 into lumen 897 and tightening coupling screws 892A and 892B to thereby electrically connect leads 860 and 870. In the illustrative case of FIGS. 8A-8C, contact pin 894 is inserted into lumen 897 such that coupling screw 892A surrounds a portion of ring contact 876A and coupling screw 892B surrounds a portion of ring contact 876B. Coupling screw 892A may be tightened via screw access 894A to constrict around contact 876A and thereby electrically connect to ring contact 876A. Similarly, coupling screw 892B may be tightened via screw access 894B to constrict around ring contact 876B and thereby electrically connect to ring contact 876B. After tightening coupling screws 892A and 892B, leads 860 and 870 are electrically connected via screw conductor 890. In alternative cases, coupling screws of female screw connector 890B may be configured to penetrate insulation of male connector 890A to thereby electrically connect to respective conductors of lead 870.

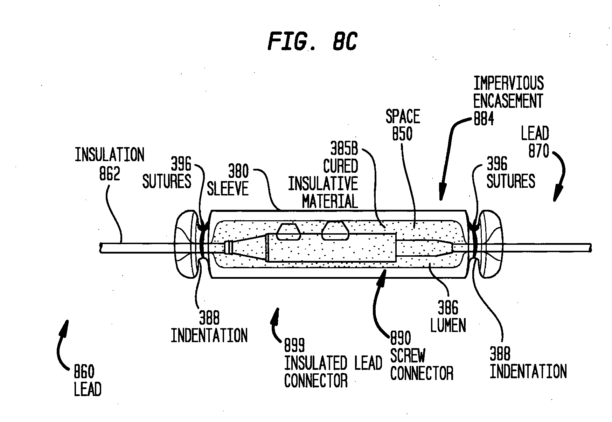

Referring to FIGS. 8B and 8C, after electrically connecting male and female screw connectors 890A and 890B, a sleeve 380, as described above, is longitudinally displaced along lead 860 or 870 until it is positioned around and encasing screw connector 890, as illustrated in FIG. 8C. In certain cases, sleeve 380 will also cover portions of leads 860 and 870 extending from male and female screw connectors 890A and 890B. As shown in FIG. 8C, the diameter of lumen 386 is large enough that, once sleeve 380 is positioned around screw connector 890, a space 850 is present between an inner surface of sleeve 380 and an outer surface of screw connector 890. Space 850 disposed within sleeve 380 is then filled with an electrically insulative material, such as one of the electrically insulative materials described above in relation to FIGS. 3A-3D. While filling space 850, the insulative material conforms around screw connector 890 and other portions of leads 860 and 870 disposed in sleeve 380. As described above, the insulative material may be a curable insulative material. When a curable insulative material is used, after filling space 850 with the curable insulative material, the curable insulative material is cured using any suitable means in order to form an ionically impervious encasement 884 and to thereby form an implantable electrically insulated lead connector 899. In the illustrative case of FIG. 8C, implantable insulated lead connector 899, comprises screw connector 890 and an ionically impervious encasement 884, which is disposed around screw connector 890. Impervious encasement 884 includes sleeve 380 and cured electrically insulative material 385B. As described above in relation to impervious encasement 384, impervious encasement 884 substantially prevents the ingress of electrically conductive body fluid and tissue to prevent the formation of any substantial conductive path of body fluid and/or tissue from screw connector 890 out of impervious encasement 884, and thereby electrically insulates screw connector 890.

In certain cases, sealing elements may be applied to sleeve 380, as described above in relation to FIGS. 3A-3D. In the illustrative case of FIG. 8C, sleeve 380 is filled with a curable electrically insulative material that is subsequently cured and is then secured with sutures 396 to form implantable insulated lead connector 899. As illustrated in FIG. 8C, sutures 396 compress sleeve 380 to leads 860 and 870 to secure sleeve 380 to leads 860 and 870. Alternatively, other sealing elements, such as O-rings and/or toroidal springs may be used to secure sleeve 380 to leads 860 and 870 to further seal lumen 386. In other cases, implantable insulated lead connector 899 is formed without applying any sealing element to sleeve 380.

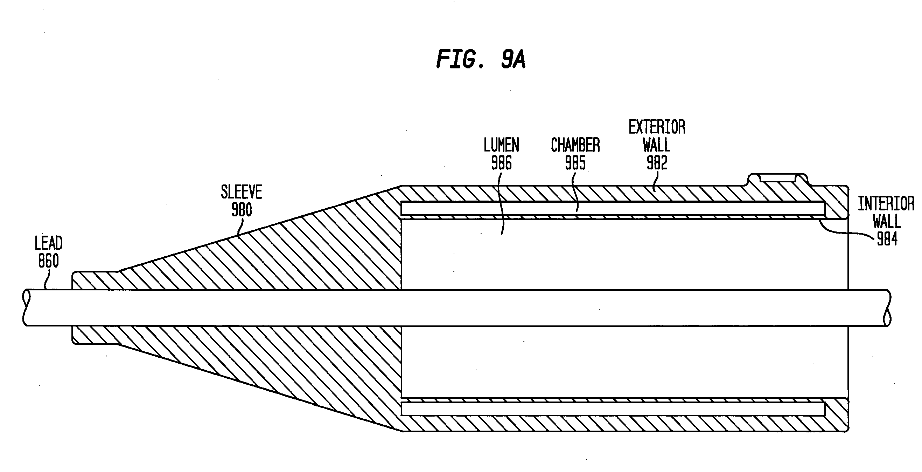

FIGS. 9A-9G illustrate implantable electrically insulated lead connector 999 in accordance with an embodiment of the present invention. In the embodiment illustrated in FIGS. 9A-9G, lead connector 999 includes a sleeve 980 defining a lumen 986. Sleeve 980 has an exterior wall 982 and a deformable interior wall 984 spaced from exterior wall 982 to form a chamber 985 between walls 982 and 984. In certain embodiments, interior wall 984 is distensible inwardly into lumen 986, as described further below.

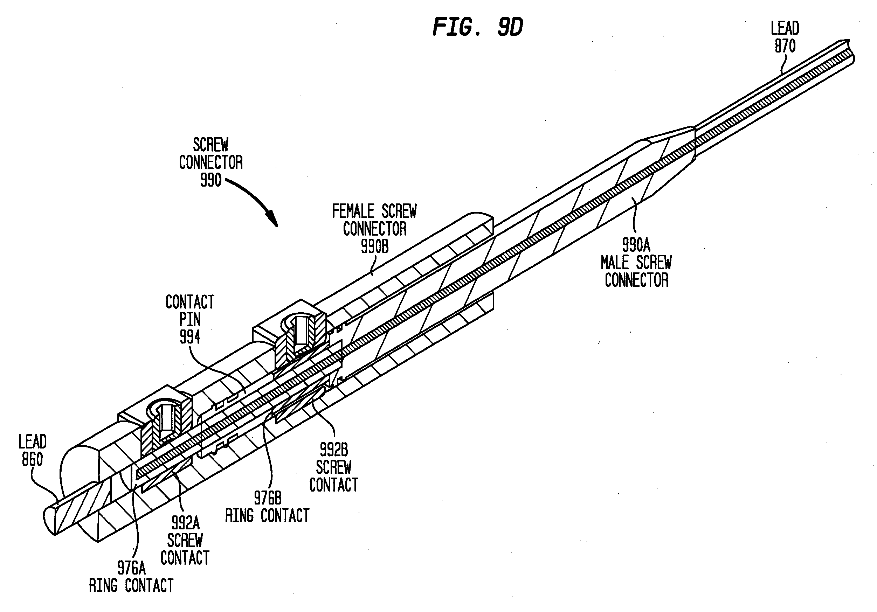

Lead connector 999 also includes a screw connector 990, shown in FIG. 9D, similar to screw connector 890 described above with reference to FIGS. 8A-8C. In certain embodiments, as illustrated in FIGS. 9D-9F, screw connector 990 includes male and female screw connectors 990A and 990B.

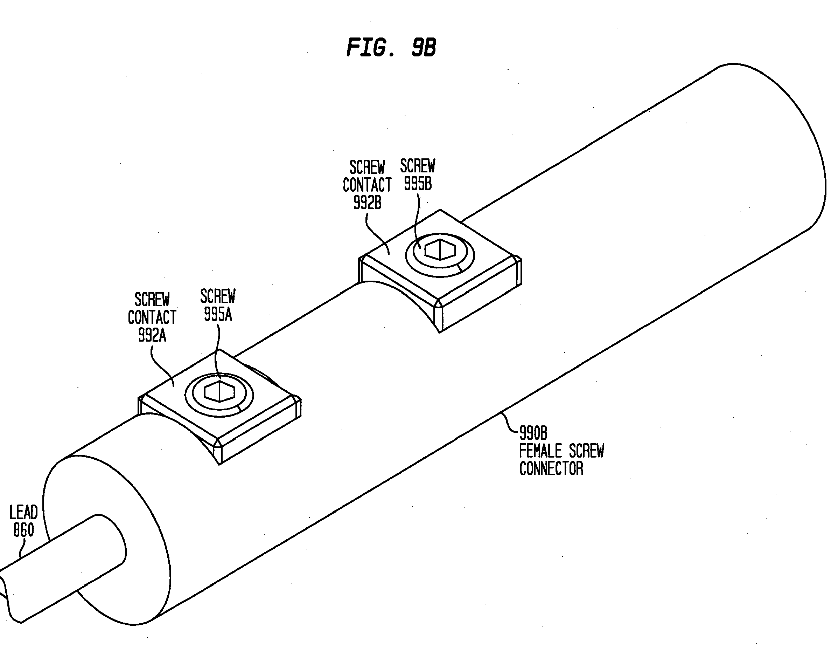

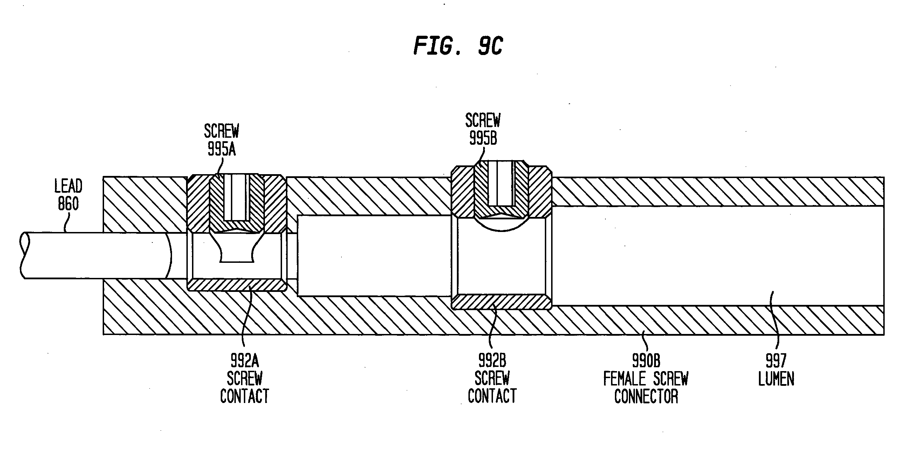

In some embodiments, as shown in FIGS. 9B and 9C, female screw connector 990B is disposed at a distal end of a lead 860 that includes one or more electrical conductors (not shown), as described above in relation to FIGS. 8A-8C. Female screw connector 990B includes a lumen 997 and a plurality of screw contacts 992A and 992B partially exposed inside of lumen 997. In the embodiment illustrated in FIGS. 9A-9G, screw contacts 992A and 992B include screws 995A and 995B. A screw contact 992 and a screw 995 may together be referred to as a coupling screw herein. In certain embodiments, as shown in FIG. 9D, male screw connector 990A is disposed at a distal end of a lead 870 that includes one or more conductors as described above in relation to FIGS. 8A-8C. Male screw connector 990A includes a contact pin 994 having first and second ring contacts 976A and 976B electrically connected to respective electrical conductors of lead 870, as described above in relation to FIGS. 8A-8C. In certain embodiments, the respective electrical conductors of lead 870 are electrically isolated from one another.

In the embodiment illustrated in FIGS. 9A-9G, one or more electrical conductors of lead 860 may be electrically connected to one or more electrical conductors of lead 870 by inserting male screw connector 990A at least partially into lumen 997 of female screw connector 990B. In certain embodiments, male screw connector 990A is inserted into lumen 997 such that ring contacts 976A and 976B of contact pin 994 are electrically connected to screw contacts 992A and 992B, respectively. In some embodiments, after inserting contact pin 994, screws 995A and 995B may be screwed in toward contact pin 994 to secure ring contacts 976A and 976B to screw contacts 992A and 992B, respectively. Screwing in a screw 995 may be referred to herein as tightening a coupling screw. In the embodiment illustrated in FIGS. 9A-9G, screw connector 990 is a two-pole electrical connector by which two electrical conductors of lead 860 are connected to two electrical conductors of lead 870 via two screw contacts 992 and two ring contacts 976. In alternative embodiments, any number of electrical conductors of respective leads may be electrically connected using a corresponding number of screw contacts and ring contacts.

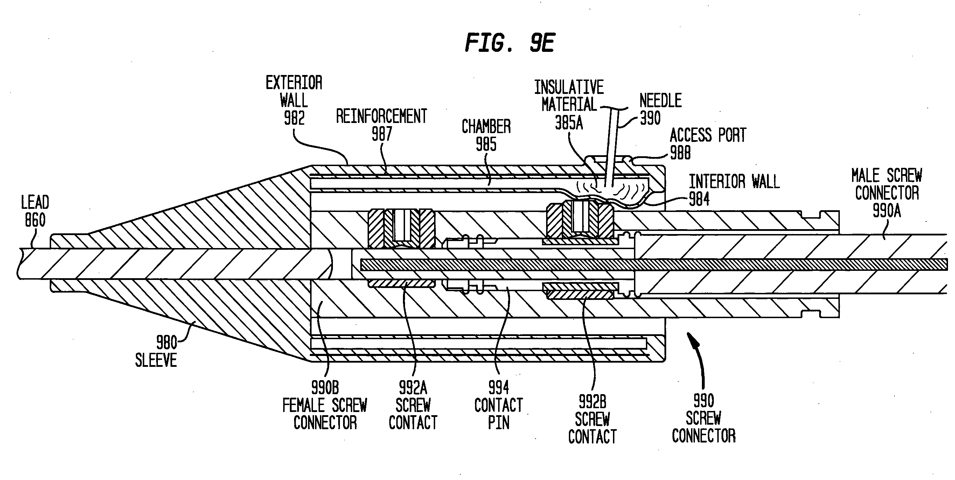

In certain embodiments, sleeve 980 is configured to be longitudinally displaced along lead 860, as described above in relation to sleeve 380. In such embodiments, sleeve 980 may be displaced along lead 860 such that the sleeve does not cover female screw connector 990B when contact pin 994 is inserted into lumen 997 of female 990B to allow access to screws 995A and 995B. In certain embodiments, once contact pin 994 has been inserted into lumen 997 and secured with screws 995, if desired, sleeve 980 may be displaced along lead 860 such that it circumferentially surrounds a portion of screw connector 990, including portions of male and female screw connectors 990A and 990B, as shown in FIG. 9E. In some embodiments, as shown in FIG. 9E, sleeve 980 may be displaced such that contact pin 994 and screw contact 992A and 992B are disposed in lumen 986 of sleeve 980. In certain embodiments, sleeve 980 surrounds an entire circumference of any portion of screw connector 990 disposed in lumen 986. In other embodiments, sleeve 980 may surround less than all of a circumference of a portion of screw connector 990 disposed in lumen 986.

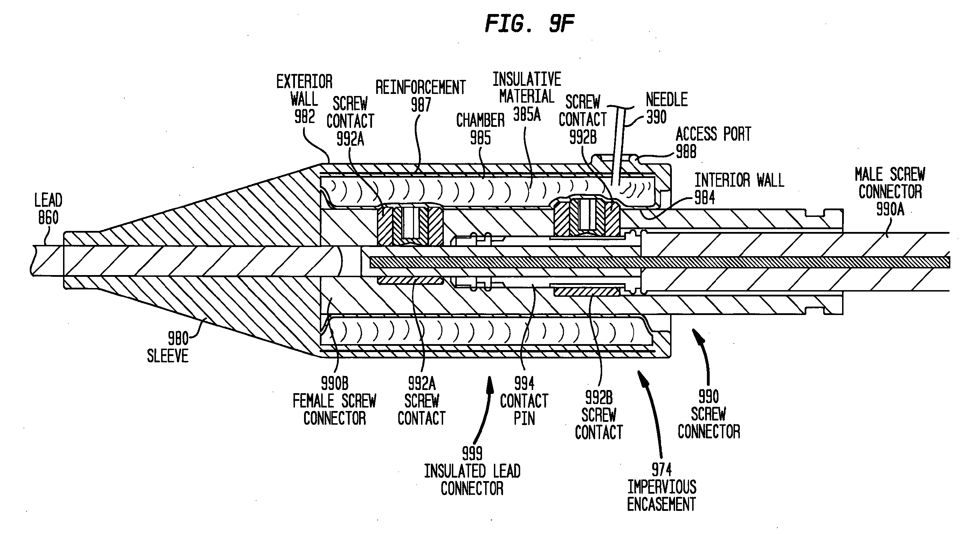

As shown in FIGS. 9E and 9F, an electrically insulative material 385A, such as one of the insulative materials described above in relation to FIGS. 3A-3D, may be injected into chamber 985 of sleeve 980. Chamber 985 is an enclosure configured to contain insulative material injected into it and which has no opening apart from a permanent or temporary opening through which the electrically insulative material is injected. As noted, interior wall 984 is deformable. As a result, interior wall 984 is distensible inwardly into lumen 986 in response to suitable forces applied by insulative material 385A in chamber 985. In certain embodiments, forces applied by insulative material 385A may result in compressive forces that cause interior wall 984 to deform, as shown in FIGS. 9E and 9F. The forces applied by insulative material 385A may be caused by the pressure with which chamber 985 is filled with insulative material 385A. In embodiments in which insulative material 385A is curable, the forces applied by insulative material 385A may be generated by the expansive forces generated by the material as it cures. In some embodiments, as shown in FIGS. 9E and 9F, the forces applied by insulative material 385A may result in compressive forces that cause interior wall 984 to press against screw connector 990. In certain embodiments, interior wall 984 may be inwardly distended with sufficient force to seal certain portions of screw connector 990 against which interior wall 984 presses. In such embodiments, the seal may resist the passage of electrically conductive moisture, such as bodily fluid or tissue to portion(s) of screw connector 990 encased by interior wall 984. In some embodiments, when interior wall 984 is inwardly distended with sufficient force to seal certain portions of screw connector 990 against which interior wall 984 presses, an impervious encasement 974 configured to electrically insulate portion(s) of screw connector 990 is formed. In certain embodiments, impervious encasement 974 includes sleeve 980 and electrically insulative material disposed in chamber 985 of sleeve 980.

Exterior wall 982 has more structural integrity than interior wall 984. In some embodiments, exterior wall 982 has sufficient structural integrity to prevent substantial deformation in response to forces applied by insulative material 385A that cause interior wall 984 to deform. The difference in structural integrity may be provided by the use of different materials, different wall thicknesses and/or the use of reinforcement structure(s). In certain embodiments, exterior wall 982 is formed of a more rigid material than interior wall 984. For example, in some embodiments, interior wall 984 may be formed of a polymer, such as silicone, while exterior wall 982 is formed of a more rigid material, such as ceramic. In alternative embodiments, exterior wall 982 may be formed of a first polymer having a greater rigidity, hardness or durometer, than a second polymer from which inwardly distensible interior wall 984 is formed. In certain embodiments, the polymer may be an elastomeric material, a polyurethane, etc. In alternative embodiments, other insulating materials may be used instead of the polymer. In other embodiments, interior and exterior walls 984 and 982 may be formed of the same material, while exterior wall 982 has a greater thickness of the material to thereby provide an exterior wall 982 with greater structural integrity than interior wall 984. In other embodiments, the greater structural integrity of exterior wall 982 may be provided by a reinforcement structure 987 embedded in exterior wall 982, as shown in FIGS. 9E and 9F. In certain embodiments, reinforcement structure 987 may be a metal or polymer structure, such as a mesh, coil, braid, or any other suitable arrangement or configuration of the material. In some embodiments, reinforcement structure 987 may be formed of a polyester fabric, such as DACRON(R). In alternative embodiments, reinforcement structure 987 may be disposed on the outside of exterior wall 982 rather than being embedded in exterior wall 982. In embodiments including a reinforcement structure 987 as described above, each of interior and exterior walls 984 and 982 may be formed of an elastomeric material.

In the embodiment illustrated in FIG. 9F, interior wall 984 is inwardly distensible such that interior wall 984 can conform to and completely encase the portions of screw contacts 992A and 992B exposed in the exterior wall 982 of female screw connector 990B. In such embodiments, interior wall 984 can electrically isolate screw contacts 992A and 992B from one - another and seal screw contacts 992A and 992B to resist the passage of moisture to either of screw contacts 992A and 992B. In some embodiments, when interior wall 984 is distended to electrically isolate and seal at least screw contacts 992, sleeve 980 forms an impervious encasement insulating portion(s) of screw connector 990. In certain embodiments, by electrically isolating and resisting the passage of moisture to screw contacts 992A and 992B, interior wall 984 in its distended state is able to substantially prevent the occurrence of a short circuit between screw contacts 992 and to substantially prevent the creation of an electrical pathway from one of screw contacts 992 into the surrounding environment by the moisture. Additionally, in some embodiments, the ingress of electrically conductive substances through sleeve 980 by osmosis may be resisted by electrically insulative material 385A disposed in chamber 985. In certain embodiments, insulative material 385A in chamber 985 may also protect screw connector 990 from external mechanical forces and impacts.

In the embodiment illustrated in FIGS. 9A-9G, chamber 985 circumferentially surrounds lumen 986. In such embodiments, interior wall 984 is inwardly distensible such that it presses against the circumference of substantially all of the portion of screw connector 990 disposed in lumen 986, as shown in FIG. 9F. In certain embodiments, as shown in FIG. 9F, interior wall 984 is inwardly distended by insulative material 385A such that interior wall 984 substantially conforms to the outer surface of the portion of screw connector 990 disposed in lumen 986. In alternative embodiments, chamber 985 may extend around less than all of the circumference of lumen 986. In some embodiments, chamber 985 may extend around only a portion of lumen 986 configured to be aligned with the exposed portions of screw contact 992 such that interior wall 984 presses against and seals little more than the exposed portions of screw contacts 992 of screw connector 990. In the embodiment illustrated in FIGS. 9A-9G, sleeve 980 includes a single chamber 985. In other embodiments, chamber 985 may be divided into two or more separate chambers that are not in fluidic communication. In such embodiments, each of the separate chambers is separately filled with insulative material.

In certain embodiments, electrically insulative material 385A may be a fluent compound. In some embodiments, the fluent compound may be liquid silicone. In certain embodiments, the fluent compound may be curable in-situ in response to heat or moisture. In such embodiments, insulative material may be cured once injected into chamber 985. In some embodiments, the fluent compound may comprise two or more compounds that begin to cure when mixed. In certain embodiments, insulative material 385A may be cured as a solid or as a gel. In other embodiments, insulative material 385A may be left in chamber 985 uncured and in a liquid form. In certain embodiments, insulative material 385A is an elastomeric material. In other embodiments, insulative material 385A may be a polyurethane. In embodiments, the viscosity of the fluent insulative material 385A may vary, and the hardness of the cured insulative material 385A may vary.

In certain embodiments, as shown in FIGS. 9E and 9F, electrically insulative material 385A may be injected into chamber 985 through a needle 390 after partially inserting needle 390 into chamber 985 through an access port 988 in exterior wall 982 of sleeve 980. In some embodiments, needle 390 is configured to puncture exterior wall 982 at access port 988. In other embodiments, access port 988 may be omitted, and needle 390 may be configured to puncture exterior wall 982 in order to access chamber 985. In alternative embodiments, access port 988 may be a membrane of a material that is less rigid than exterior wall 982 and that is configured to be punctured by needle 390 to access chamber 985. In some embodiments, access port 988 includes a slit in exterior wall 982. In such embodiments, a blunt needle 390 may be used to inject insulative material 385A. In some embodiments, the slit in access port 988 may be sealed with an externally applied plug or curable insulative material after injecting insulative material 385A into chamber 985 to prevent insulative material 385A from flowing out of chamber 985 through the slit. In other embodiments, when a curable insulative material 385A is injected into chamber 985, the plug or other curable insulative material for sealing the slit in access port 988 may be omitted, as insulative material 385A may seal the slit once cured.

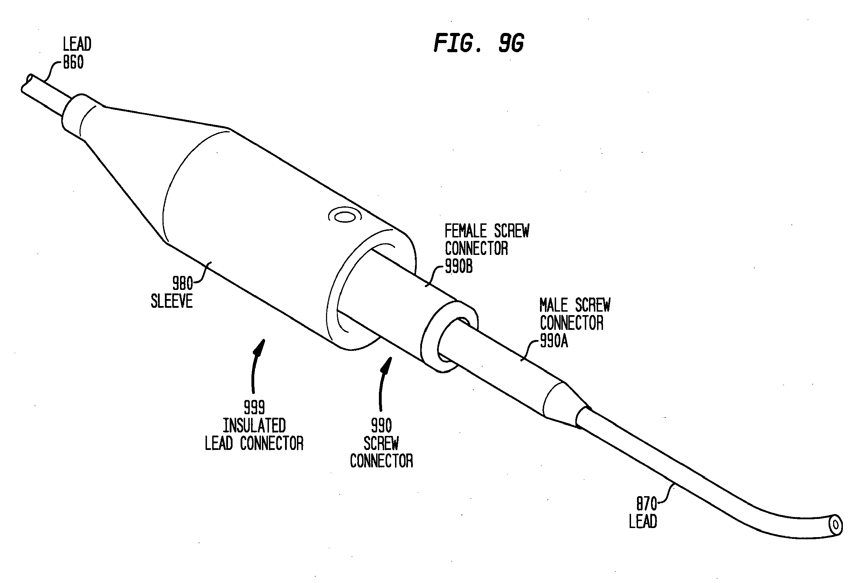

Additionally, in embodiments in which exterior wall 982 is formed of a relatively rigid material, such as ceramic, as described above, access port 988 in exterior wall 982 may be formed of a different, less rigid material to allow needle 390 to puncture or pass through a slit in exterior wall 982 at access port 988. In such embodiments, access port 988 may be formed of an elastomer, for example. In embodiments including two or more separate chambers in sleeve 980, as described above, an access port 988 may be provided for each of the separate chambers. In alternative embodiments, sleeve 980 may include a one-way valve through which insulative material 385A is injected into chamber 985 without the use of a needle. FIG. 9G is a perspective view of implantable insulated lead connector 999 in which sleeve 980 is sealing a portion of screw connector 990 disposed in the lumen 986 of sleeve 980 after injecting and curing, if desired, insulative material 385A in accordance with embodiments of the present invention, as described above.

In the embodiments described above in relation to FIGS. 9A-9G, sleeve 980 is used to seal and/or electrically insulate portion(s) of screw connector 990. In other embodiments, sleeve 980 may be used to seal and/or electrically insulate portion(s) of any one of the conductor connectors described.

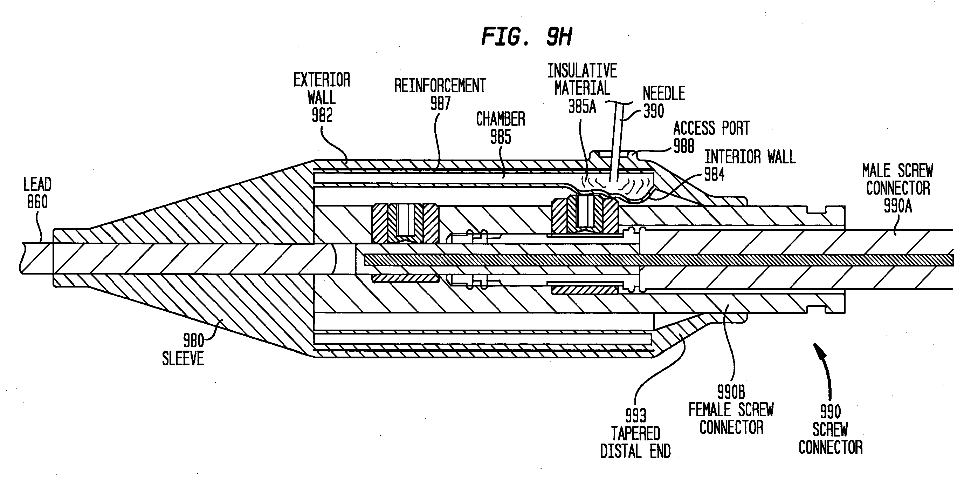

FIG. 9H illustrates an implantable insulated lead connector 999 in which sleeve 980 includes a tapered distal end 993 configured to provide an additional seal around screw connector 990. In the embodiment illustrated in FIG. 9H, tapered distal end 993 forms an interference fit around a circumference of female screw connector 990B. As shown in FIG. 9H, in certain embodiments, the interference fit may be formed prior to injecting insulative material 385A into chamber 985, as described above. In some embodiments, the seal formed by tapered distal end 993 may resist the passage of electrically conductive moisture of tissue into lumen 986 of sleeve 986. In certain embodiments, tapered distal end may include one or more external indentations similar to external indentations 388 describe above with reference to FIGS. 3A-3D. One or more sealing elements, such as sutures, O-rings or toroidal springs may be applied in the external indentation(s), as described above, to compress tapered distal end 993 to female screw connector 990B to improve the seal between tapered distal end 993 to female screw connector 990B. In certain embodiments, after injecting and curing, if desired, insulative material 385A, tapered distal end 993 and distended interior wall 984 both seal the portion of screw connector 990 disposed in lumen 986.

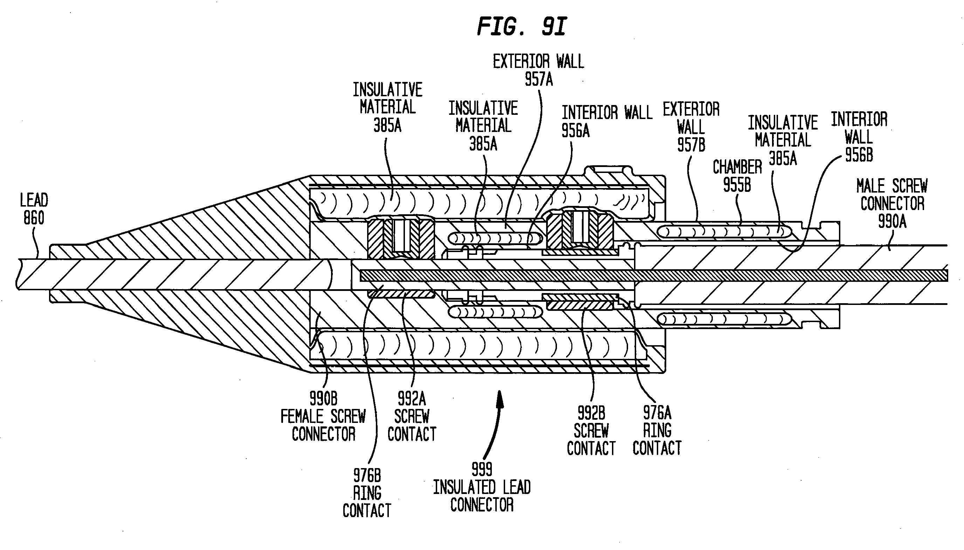

FIG. 9I illustrates an implantable insulated lead connector 999 in which female screw connector 990B includes exterior walls 957 and deformable interior walls 956 spaced from exterior walls 957 to form chambers 955 between walls 957 and 956. In the embodiment illustrated in FIG. 9I, female screw connector 990B includes chambers 955A and 955B, each of which is similar to chamber 985 of sleeve 980, as described above. Chambers 955A and 955B. surround inwardly distensible interior walls 956A and 956B, respectively. Additionally, chamber 955A is disposed between interior and exterior walls 956A and 957A and chamber 955B is disposed between interior and exterior walls 956B and 957B. Exterior walls 957 are similar to exterior walls 982 described above. Exterior walls 957 have greater structural integrity than interior walls 956. The greater structural integrity may be provided as described above in relation to exterior walls 982. In certain embodiments, each of chambers 955A may extend partially or completely around a circumference of female screw connector 990B. Each of interior walls 956A and 956B is similar to interior wall 984 and is inwardly distensible in response to forces applied by insulative material 385A disposed in chambers 955A and 955B, respectively, which, as described above, result in compressive forces applied to interior walls 956A and 956B. Electrically insulative material 385A, or a different insulative material, may be injected into chambers 955A and 955B as described above in relation to chamber 985. In the embodiment illustrated in FIG. 9I, the forces applied by insulative material 385A result in compressive forces that press interior wall 956A against contact pin 994 to improve the insulation of the electrical connection between screw contact 992A and ring contact 976A and to reduce any air pockets that may be present between interior wall 956A and contact pin 994. Similarly, the forces applied by insulative material 385A result in compressive forces that press interior wall 956B against contact pin 994 to improve the insulation of the electrical connection between screw contact 992B and ring contact 976B and to reduce any air pockets that may be present between interior wall 956B and contact pin 994. While the embodiment illustrated in FIG. 9I includes two chambers 955A and 955B, female screw connector 990B may include any number of chambers 955 in accordance with embodiments of the present invention.

As noted above, in certain embodiments of the present invention, an implantable electrically insulated lead connector may be used to form a reliable, insulated electrical connection between leads of device components of an implantable medical device. According to some embodiments, the implantable insulated lead connector may be used to create an insulated electrical connection between any two leads at nearly any location along either of the leads. Additionally, implantable insulated lead connectors according to embodiments of the present invention may be used to repair leads connecting distributed components of an implantable medical device.

In certain embodiments described above, the insulative material is noted to be silicone. It is also noted that in certain embodiments the insulative material may be a curable insulative material. Also, in some embodiments described above, the sleeve may be at least partially formed of silicone. As one of ordinary skill in the art would appreciate, many silicones used in medical device applications are fluid resistant, not fluid impermeable. Rather, many silicones are ion impermeable; that is, they are impermeable to electrically conductive fluid. As such, as used herein, the term impervious encasement means an encasement that is impermeable to ionic or electrically conductive substances, such as electrically conductive fluid or tissue.

## Claims

1. An encasement sealable to be impervious configured to electrically insulate a conductor connector (990) configured to electrically connect distal ends of first and second leads (860, 870) electrically connectable to first and second device components (235, 237) of an implantable medical device (120), comprising:
a sleeve (980), configured to circumferentially surround the conductor connector (990), having an exterior wall (982) and a deformable interior wall (984) spaced to define a chamber (985) therebetween, **characterized in that** the exterior wall has greater stuctured integrity then the interior wall.

2. The encasement of claim 1, wherein the interior wall (984) defines a lumen (986) of the sleeve (980) configured to receive at least a portion of the conductor connector (990).

3. The encasement of claim 2, wherein the interior wall (984) is configured to distend inwardly into the lumen (986) in response to force applied by an electrically insulative material (985a) in the chamber (985).

4. The encasement of claim 2, wherein the sleeve (980) is configured to extend around less than all of a circumference of the conductor connector (990) when the conductor connector (990) is disposed in the lumen (986).

5. The encasement of claim 2, wherein the chamber (985) is configured to extend around less than all of a circumference of the conductor connector (990) when the conductor connector (990) is disposed in the lumen (986).

6. The encasement of claim 2, wherein the sleeve (980) includes a tapered distal end configured to form an interference fit around a circumference of the conductor connector (990) when the conductor connector (990) is disposed in the lumen (986).

7. The encasement of claim 6, wherein the tapered distal end includes one or more external indentations configured to receive one or more sealing elements configured to compress the tapered distal end to the conductor connector (990).

8. The encasement of claim 1, wherein the sleeve (980) includes a plurality of separate chambers that are not in fluidic communication with one another.

9. The encasement of claim 1, wherein the chamber (985) is configured to receive electrically insulative material (985a) via an access port (988) included in the exterior wall (982).

10. A non-surgical method of connecting leads (860, 870) of implantable medical device components, outside of a recipient's body comprising first and second implantable components (235, 237) having first and second leads (860, 870), respectively, the method comprising:
electrically connecting distal ends of the first and second leads (860, 870) with a conductor connector (990);
longitudinally displacing a sleeve (980) along the first lead (960) such that the sleeve (980) circumferentially surrounds the conductor connector (990), wherein the sleeve (980) has an exterior wall (982) and a deformable interior wall (984) spaced to define a chamber (985) therebetween, wherein the exterior wall has grether structured integrity than the interior wall; and
injecting a fluent electrically insulative material (985a) into the chamber (985) such that the insulative material (985a) causes the interior wall (985) to distend inwardly.

11. The non-surgical method of claim 10, wherein said injecting the fluent electrically insulative material (985a) into the chamber (985) comprises:
inserting a needle (390) into the chamber (985) via an access port (988) in the exterior wall (982); and
injecting the insulative material (985a) into the chamber (985) through the needle (390).

12. The non-surgical method of claim 11, wherein said inserting the needle (390) into the chamber (985) via an access port (988) in the exterior wall (982) comprises:
inserting the needle (390) into the chamber (985) via a slit in the access port (988).

13. The non-surgical method of claim 12, further comprising:
sealing the slit after removing the needle (390) from the slit.

14. The non-surgical method of claim 10 wherein said injecting the fluent electrically insulative material (985a) into the chamber (985) comprises:
puncturing the exterior wall (982) with a needle (390) to insert the needle (390) into the chamber (985); and
injecting the insulative material (985a) into the chamber (985) through the needle (390).

## Patentansprüche

1. Gehäuse, das so abgedichtet werden kann, dass es undurchlässig ist, und so eingerichtet ist, dass es einen Leiter-Verbinder (990) elektrisch isoliert, der so eingerichtet ist, dass er vordere Enden einer ersten und einer zweiten Zuleitung (860, 870) elektrisch verbindet, die elektrisch mit einer ersten und einer zweiten Vorrichtungs-Komponente (235, 237) einer implantierbaren medizinischen Vorrichtung (120) verbunden werden können, wobei es umfasst:
eine Hülse (980), die so eingerichtet ist, dass sie den Leiter-Verbinder (990) am Umfang umgibt, und die eine Außenwand (982) sowie eine verformbare Innenwand (984) aufweist, die voneinander beabstandet sind, so dass eine Kammer (985) zwischen ihnen gebildet wird, **dadurch gekennzeichnet, dass** die Außenwand größere strukturelle Integrität hat als die Innenwand.

2. Gehäuse nach Anspruch 1, wobei die Innenwand (984) einen Hohlraum (986) der Hülse (980) umgrenzt, der zum Aufnehmen wenigstens eines Abschnitts des Leiter-Verbinders (990) eingerichtet ist.

3. Gehäuse nach Anspruch 1, wobei die Innenwand (984) so eingerichtet ist, dass sie sich in Reaktion auf eine Kraft, die durch ein elektrisch isolierendes Material (985a) in der Kammer (985) ausgeübt wird, nach innen in den Hohlraum (986) hinein ausdehnt.

4. Gehäuse nach Anspruch 2, wobei die Hülse (980) so eingerichtet ist, dass sie sich um weniger als einen gesamten Umfang des Leiter-Verbinders (990) herum erstreckt, wenn der Leiter-Verbinder (990) in dem Hohlraum (986) angeordnet ist.

5. Gehäuse nach Anspruch 2, wobei die Kammer (985) so eingerichtet ist, dass sie sich um weniger als einen gesamten Umfang des Leiter-Verbinders (990) herum erstreckt, wenn der Leiter-Verbinder (990) in dem Hohlraum (986) angeordnet ist.

6. Gehäuse nach Anspruch 2, wobei die Hülse (980) ein konisches vorderes Ende enthält, das so eingerichtet ist, dass es eine Presspassung um einen Umfang des Leiter-Verbinders (990) herum erzeugt, wenn der Leiter-Verbinder (990) in dem Hohlraum (986) angeordnet ist.

7. Gehäuse nach Anspruch 6, wobei das konische vordere Ende eine oder mehrere äußere Vertiefung/en enthält, die so eingerichtet ist/sind, dass sie ein oder mehrere Dichtungselement/e aufnimmt/aufnehmen, das/die so eingerichtet ist/sind, dass es/sie das konische vordere Ende des Leiter-Verbinders (990) zusammendrückt/zusammendrücken.

8. Gehäuse nach Anspruch 1, wobei die Hülse (980) eine Vielzahl separater Kammern enthält, die nicht in Fluidverbindung miteinander stehen.

9. Gehäuse nach Anspruch 1, wobei die Kammer (985) so eingerichtet ist, dass sie elektrisch isolierendes Material (985a) über eine Zugangsöffnung (988) aufnimmt, die in der äußeren Wand (982) enthalten ist.

10. Nicht-invasives Verfahren zum Verbinden von Zuleitungen (860, 870) von Komponenten einer implantierbaren medizinischen Vorrichtung außerhalb des Körpers eines Patienten, die eine erste und eine zweite implantierbare Komponente (235, 237) umfasst, die eine erste bzw. eine zweite Zuleitung (860, 870) aufweisen, wobei das Verfahren umfasst:
elektrisches Verbinden vorderer Enden der ersten und der zweiten Zuleitung (860, 870) mit einem Leiter-Verbinder (990);
Verschieben einer Hülse (980) entlang der ersten Zuleitung (960) in Längsrichtung, so dass die Hülse (980) den Leiter-Verbinder (990) am Umfang umgibt, wobei die Hülse (980) eine Außenwand (982) sowie eine verformbare Innenwand (984) aufweist, die beabstandet sind, so dass eine Kammer (985) zwischen ihnen gebildet wird, und die äußere Wand größere strukturelle Integrität hat als die Innenwand; und
Einspritzen eines flüssigen elektrisch isolierenden Materials (985a) in die Kammer (985), so dass das isolierende Material (985a) bewirkt, dass sich die Innenwand (985) nach innen ausdehnt.

11. Nicht-invasives Verfahren nach Anspruch 10, wobei das Einspritzen des flüssigen elektrisch isolierenden Materials (985a) in die Kammer (985) umfasst:
Einführen einer Nadel (390) in die Kammer (985) über eine Zugangsöffnung (988) in der Außenwand (982); und
Einspritzen des isolierenden Materials (985a) in die Kammer (985) über die Nadel (390).

12. Nicht-invasives Verfahren nach Anspruch 11, wobei das Einführen der Nadel (390) in die Kammer (985) über eine Zugangsöffnung (988) in der Außenwand (982) umfasst:
Einführen der Nadel (390) in die Kammer (985) über einen Schlitz in der Zugangsöffnung (988).

13. Nicht-invasives Verfahren nach Anspruch 12, das umfasst:
Abdichten des Schlitzes nach Entfernen der Nadel (390) aus dem Schlitz.

14. Nicht-invasives Verfahren nach Anspruch 10, wobei Einspritzen des flüssigen elektrisch isolierenden Materials (985a) in die Kammer (985) umfasst:
Durchstechen der äußeren Wand (982) mit einer Nadel (390), um die Nadel (390) in die Kammer (985) einzuführen; und
Einspritzen des isolierenden Materials (985a) in die Kammer (985) über die Nadel (390).

## Revendications

1. Enveloppe pouvant être scellée pour être hermétique, configurée pour isoler électriquement un connecteur de conducteurs (990) configuré pour relier électriquement les extrémités distales de première et seconde sondes (860, 870) pouvant être reliées électriquement à des premier et second composants de dispositif (235, 237) appartenant à un dispositif médical implantable (120), comprenant :
un manchon (980) configuré pour entourer circonférentiellement le connecteur de conducteurs (990), comportant une paroi extérieure (982) et une paroi intérieure déformable (984) espacées pour définir une chambre (985) entre elles, **caractérisées en ce que** la paroi extérieure présente une intégrité de structure plus grande que la paroi intérieure.

2. Enveloppe selon la revendication 1, dans laquelle la paroi intérieure (984) définit une lumière (986) du manchon (980) configurée pour recevoir au moins une partie du connecteur de conducteurs (990).

3. Enveloppe selon la revendication 2, dans laquelle la paroi intérieure (984) est configurée pour se détendre vers l'intérieur dans la lumière (986) en réponse à une force appliquée par un matériau isolant électriquement (985a) dans la chambre (985).

4. Enveloppe selon la revendication 2, dans laquelle le manchon (980) est configuré pour s'étendre autour de moins de la totalité de la circonférence du connecteur de conducteurs (990) lorsque le connecteur de conducteurs (990) est disposé dans la lumière (986).

5. Enveloppe selon la revendication 2, dans laquelle la chambre (985) est configurée pour s'étendre autour de moins de la totalité de la circonférence du connecteur de conducteurs (990) lorsque le connecteur de conducteurs (990) est disposé dans la lumière (986).

6. Enveloppe selon la revendication 2, dans laquelle le manchon (980) inclut une extrémité distale effilée configurée pour former un joint à ajustement serré autour de la circonférence du connecteur de conducteurs (990) lorsque le connecteur de conducteurs (990) est disposé dans la lumière (986).

7. Enveloppe selon la revendication 6, dans laquelle l'extrémité distale effilée inclut un ou plusieurs renfoncements configurés pour recevoir un ou plusieurs éléments de scellement configurés pour comprimer l'extrémité distale effilée sur le connecteur de conducteurs (990).

8. Enveloppe selon la revendication 1, dans laquelle le manchon (980) inclut une pluralité de chambres séparées qui ne sont pas en communication par fluide l'une avec l'autre.

9. Enveloppe selon la revendication 1, dans laquelle la chambre (985) est configurée pour recevoir un matériau isolant électriquement (985a) par l'intermédiaire d'un orifice d'accès (988) inclus dans la paroi extérieure (982).

10. Procédé non chirurgical de connexion de sondes (860, 870) de composants d'un dispositif médical implantable extérieur au corps d'un receveur, comprenant des premier et second composants implantables (235, 237) comportant respectivement des première et seconde sondes (860, 870), le procédé comprenant :
la connexion électrique d'extrémités distales des première et seconde sondes (860, 870) avec un connecteur de conducteurs (990),
le déplacement longitudinal d'un manchon (980) le long de la première sonde (960) de telle sorte que le manchon (980) entoure circonférentiellement le connecteur de conducteurs (990), le manchon (980) comportant une paroi extérieure (982) et une paroi intérieure déformable (984) espacées pour définir une chambre (985) entre elles, la paroi extérieure présentant une intégrité de structure plus grande que la paroi intérieure, et
l'injection d'un matériau fluide isolant électriquement (985a) dans la chambre (985) de telle sorte que le matériau isolant (985a) amène la paroi intérieure (985) à se détendre vers l'intérieur.

11. Procédé non chirurgical selon la revendication 10, dans lequel ladite injection du matériau fluide isolant électriquement (985a) dans la chambre (985) comprend :
l'insertion d'une aiguille (390) dans la chambre (985) par l'intermédiaire d'un orifice d'accès (988) ménagé dans la paroi extérieure (982), et
l'injection du matériau isolant (985a) dans la chambre (985) par l'intermédiaire de l'aiguille (390).

12. Procédé non chirurgical selon la revendication 11, dans lequel ladite insertion de l'aiguille (390) dans la chambre (985) par l'intermédiaire d'un orifice d'accès (988) ménagé dans la paroi extérieure (982) comprend :
l'insertion de l'aiguille (390) dans la chambre (985) par l'intermédiaire d'une fente pratiquée dans l'orifice d'accès (988).

13. Procédé non chirurgical selon la revendication 12, comprenant en outre :
le scellement de la fente après avoir retiré l'aiguille (390) de la fente.

14. Procédé non chirurgical selon la revendication 10, dans lequel ladite injection du matériau fluide isolant électriquement (985a) dans la chambre (985) comprend :
la perforation de la paroi extérieure (982) avec une aiguille (390) afin d'insérer l'aiguille (390) dans la chambre (985), et
l'injection du matériau isolant (985a) dans la chambre (985) par l'intermédiaire de l'aiguille (390).
